# EUROPEAN PATENT APPLICATION

(11) **EP 4 261 288 A1**
(43) Date of publication of application: **18.10.2023**
(21) Application number: 22167725.5
(22) Date of filing: 11.04.2022
(51) Int. Cl.: C12P 7/42, C12P 19/32, C12P 41/00, C12P 7/62

(54) **METHOD FOR PREPARING ALPHA-BRANCHED BETA -HYDROXY CARBONYL COMPOUNDS BY ENZYMATIC-CATALYZED REDUCTIVE ALDOL REACTION**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a method for preparing α-branched β'-hydroxy carbonyl compounds through enzymatic-catalyzed reductive aldol reaction by reacting α,β-unsaturated carbonyl donors with carbonyl acceptors in the presence of a polypeptide capable of catalyzing reductive aldol reactions and a cofactor, wherein the polypeptide is an enoyl-CoA carboxylase/reductase (Ecr). The replacement of the native CO₂ electrophile in enoyl-CoA carboxylases/reductases (Ecrs) by different carbonyl acceptors advantageously creates a new-to-nature biocatalytic route towards α-branched β'-hydroxy carbonyl compounds.

## Description

The present invention relates to a method for preparing α-branched β'-hydroxy carbonyl compounds through enzymatic-catalyzed reductive aldol reaction by reacting α,β-unsaturated carbonyl donors with carbonyl acceptors in the presence of a polypeptide capable of catalyzing reductive aldol reactions and a cofactor, wherein the polypeptide is an enoyl-CoA carboxylase/reductase (Ecr). The replacement of the native CO₂ electrophile in enoyl-CoA carboxylases/reductases (Ecrs) by different carbonyl acceptors advantageously creates a new-to-nature biocatalytic route towards α-branched β'-hydroxy carbonyl compounds.

### Background of the invention

C-C bond forming reactions allow the synthesis of complex molecules from simpler precursors and are a cornerstone in chemistry and biology. Among the most important C-C forming reactions are aldol reactions through which two carbonyl compounds are converted into a β-hydroxy functionalized carbonyl product. Many biologically relevant compounds display this structural element in their backbone, which makes these molecules highly relevant targets for organic synthesis. Consequently, a rich aldol chemistry has been developed to access β-hydroxy functionalized carbonyl molecules in a stereo- and regioselective manner.

In nature, C-C bond formations are catalyzed by aldolases, which provide an interesting alternative to purely synthetic chemical routes, because these enzymes can operate under mild conditions at high turnover rates and with exquisite diastereo- and enantioselectivity. Over the last years, various naturally occurring aldolases have been successfully evolved for improved activity under different conditions and engineered for increased promiscuity past their natural substrates for diverse applications in biosynthesis and organo-catalysis.

More than 30 years ago, reductive aldol reactions were introduced to organic chemistry by Revis and Hilty. These reactions enable the direct, catalytic coupling of α,β-unsaturated carbonyl compounds, such as enoates or acrylates with carbonyl electrophiles, which has greatly extended the scope of C-C bond forming reactions. However, several challenges remain for application of these reactions in synthetic chemistry. The majority of reductive aldol reactions involve hydrosilanes as reductants which are relatively expensive and lead to unwanted adducts that incorporate silyl ether moieties. Other approaches employ precious and scarce transition metals as catalysts, which allow diastereomer- and enantioselective C-C couplings, but require enantiomerically pure organo ligands that are difficult to synthesize. In principle, above challenges could be overcome by the availability of suitable biocatalysts that would enable stereoselective reductive aldol reactions under mild and sustainable reaction conditions. However, so far, no enzyme has been described that is able to catalyze a reductive aldol reaction.

Thus, it is the objective of the present invention to provide a method for preparing α-branched β'-hydroxy carbonyl compounds by reductive aldol reaction with the use of a biocatalyst that enables stereoselective reductive aldol reactions under mild and sustainable reaction conditions. Thus, it is the objective of the present invention to provide a method for preparing α-branched β'-hydroxy carbonyl compounds by enzymatic-catalyzed reductive aldol reaction.

The objective of the present invention is solved by the teaching of the independent claims. Further advantageous features, aspects and details of the invention are evident from the dependent claims, the description, the figures, and the examples of the present application.

### Brief description of the invention

Surprisingly it has been found that a method for preparing an α-branched β'-hydroxy carbonyl compound by reductive aldol reaction, comprising performing an enzymatic-catalyzed reductive aldol reaction with an α,β-unsaturated carbonyl donor, wherein the α,β-unsaturated carbonyl donor is a coenzyme A thioester of an α,β-unsaturated carboxylic acid, and a carbonyl acceptor, wherein the carbonyl acceptor is an aldehyde, by using a polypeptide capable of catalyzing reductive aldol reactions in the presence of a cofactor, wherein the polypeptide is an enoyl-CoA carboxylase/reductase resolves the above objective.

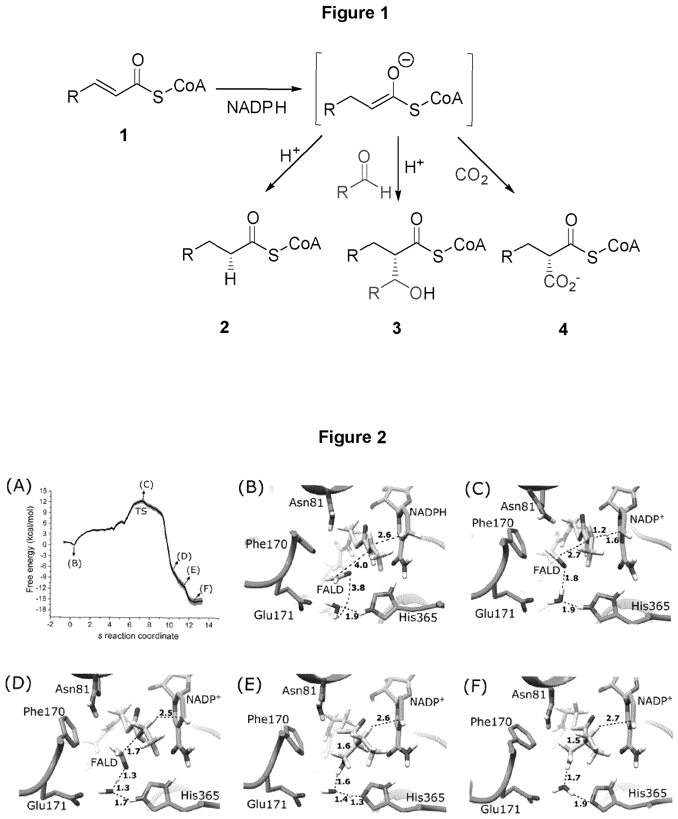

Enoyl-CoA carboxylases/reductases (Ecrs) naturally catalyze the reductive carboxylation of enoyl-CoA thioesters **1** (Fig. 1). Using NADPH as reducing equivalent, enoyl-CoA carboxylases/reductases (Ecrs) form an enolate intermediate, which is subsequently resolved by CO₂ as electrophile, yielding the corresponding (2S)-alkylmalonyl-CoA **4**. In the absence of CO₂, the enolate intermediate is protected at the active site from protonation through reversible formation of a C2-ene adduct, and slowly quenched over time by water into the corresponding alkyl-CoA **2**. The inventors of the present invention have now found that the catalytic cycle of enoyl-CoA carboxylases/reductases (Ecrs) provides a unique opportunity to intercept and reroute the enolate intermediate into other reaction paths by replacing the native CO₂ electrophile in enoyl-CoA carboxylase/reductase (Ecr) by different carbonyl acceptors to create a new-to-nature biocatalytic route towards α-branched β'-hydroxyacyl-CoAs **3**.

It has been surprisingly found that enoyl-CoA carboxylases/reductases (Ecrs) can natively catalyze the reductive aldol reaction of crotonyl-CoA and formaldehyde at a turnover frequency of 30 s⁻¹ and a yield of 96%. On the basis of this finding, the mechanistic basis of catalysis was investigated by molecular dynamics simulations which allowed improving and expanding the scope of enzyme-catalyzed reductive aldol reactions by enoyl-CoA carboxylases/ reductases (Ecrs). In consequence, enzyme engineering in combination with reaction proof-reading strategies allowed product yield increase and more advantageously enabled the synthesis of more complex compounds with two newly-formed stereocenters at high diastereomeric ratios of up to 94:6. Furthermore, by employing an engineered enoyl-CoA carboxylase/reductase (Ecr), the combinatorial synthesis of 24 different products from various CoA-thioester and aldehydes was possible.

Thus, the method of the present invention advantageously comprises the use of enoyl-CoA carboxylases/reductases (Ecrs) as biocatalysts for stereoselective reductive aldol reactions under mild and sustainable reaction conditions. The use of enoyl-CoA carboxylases/reductases (Ecrs) as biocatalysts for enzymatic-catalyzed reductive aldol reactions advantageously expands the repertoire of known enzymatic transformations by establishing reductive aldol couplings as a new-to-nature reaction with great potential for different applications in synthetic biology, biocatalysis and organic synthesis.

The enoyl-CoA carboxylase/reductase (Ecr) may be a wild type enoyl-CoA carboxylase/reductase or a modified enoyl-CoA carboxylase/reductase. In preferred embodiments, the enoyl-CoA carboxylase/reductase may be a crotonyl-CoA carboxylase/reductase (Ccr), wherein the crotonyl-CoA carboxylase/reductase (Ccr) may be a wild type crotonyl-CoA carboxylase/reductase or a modified crotonyl-CoA carboxylase/reductase. In preferred embodiments, the wild type crotonyl-CoA carboxylase/reductase may be obtained from *Kitasatospora setae* or *Caulobacter crescentus.*

Thus, the polypeptide capable of catalyzing reductive aldol reactions may be a wild type crotonyl-CoA carboxylase/reductase obtained from *Kitasatospora setae* comprising the amino acid sequence of SEQ ID NO: 1. In a further preferred embodiment, the polypeptide capable of catalyzing reductive aldol reactions may be a wild type crotonyl-CoA carboxylase/reductase obtained from *Caulobacter crescentus* comprising the amino acid sequence of SEQ ID NO: 2. Also other wild type crotonyl-CoA carboxylases/reductases from other microorganisms may be used in the method of the present invention. Especially preferred are herein also wild type or modified crotonyl-CoA carboxylases/reductases comprising at least 95% sequence identity to amino acid sequence SEQ ID NO: 1 or SEQ ID NO:2.

In further preferred embodiments, the polypeptide capable of catalyzing reductive aldol reactions may be a modified crotonyl-CoA carboxylase/reductase, wherein the modified crotonyl-CoA carboxylase/reductase may be derived from a wild type crotonyl-CoA carboxylase/reductase. In preferred embodiments, the modified crotonyl-CoA carboxylase/reductase may be derived from wild type crotonyl-CoA carboxylase/reductase from *Kitasatospora setae.* Thus, in preferred embodiments, the polypeptide capable of catalyzing reductive aldol reactions may comprise the amino acid sequence of SEQ ID NO: 19, SEQ ID NO: 20, SEQ ID NO: 21, SEQ ID NO: 22, SEQ ID NO: 23, or SEQ ID NO: 24. In further preferred embodiments, the polypeptide capable of catalyzing reductive aldol reaction may comprise the amino acid sequence of SEQ ID NO: 25, SEQ ID NO: 26 or SEQ ID NO: 27. In further preferred embodiments, the polypeptide capable of catalyzing reductive aldol reactions may be a modified crotonyl-CoA carboxylase/reductase, wherein the modified crotonyl-CoA carboxylase/reductase may be derived from wild type crotonyl-CoA carboxylase/reductase from *Caulobacter crescentus.* Thus, in further preferred embodiments, the polypeptide capable of catalyzing reductive aldol reaction may comprise the amino acid sequence of SEQ ID NO: 29.

According to the present invention, the method comprises performing an enzymatic-catalyzed reductive aldol reaction in the presence of a cofactor. Herein, a particularly preferred cofactor relates to NADPH. In preferred embodiments, wherein the enoyl-CoA carboxylase/reductase is a crotonyl-CoA carboxylase/reductase (Ccr), it is particularly preferred that the enzymatic-catalyzed reductive aldol reaction is performed in the presence of the cofactor NADPH.

The α,β-unsaturated carbonyl donor is a coenzyme A thioester of an α,β-unsaturated carboxylic acid, wherein the α,β-unsaturated carboxylic acid may be selected from acrylic acid or crotonic acid or which may comprise another β-substituents such as optionally substituted alkyl, alkenyl, alkynyl, alkoxy, carboxy, aminocarbonyl, thiocarbonyl, heteroalkyl, heteroalkenyl, heteroalkynyl, carboxyalkyl, aminoalkyl, haloalkyl, alkylthioalkyl, cycloalkyl, aryl, arylalkyl, heterocycloalkyl, heteroaryl, or heteroarylalkyl, such as, for example, *trans-*cinnamic acid, 5-chloro-2-pentenoic acid, *trans*-2-hexenoic acid, 5-methyl-2-hexenoic acid, *trans*-2-penten-4-ynoic acid and penta-2,4-dienoic acid. Particularly preferred are acrylic acid and crotonic acid, and crotonic acid is even more preferred.

The carbonyl acceptor is an aldehyde, which may be selected from formaldehyde, acetaldehyde or which may be also an aldehyde having other carbon substituents such as optionally substituted alkyl or optionally substituted aryl. Particularly preferred are formaldehyde and acetaldehyde, and formaldehyde is even more preferred.

The replacement of native CO₂ electrophile in enoyl-CoA carboxylase/reductase (Ecr) by different carbonyl acceptors advantageously provides α-branched β'-hydroxy carbonyl compounds in form of α-branched β'-hydroxyacyl-CoAs. In preferred embodiments the α-branched β'-hydroxyacyl-CoAs can be further used as valuable α-branched-β-hydroxyacyl-CoA building blocks in organic synthesis, biocatalysis and synthetic biology.

In further preferred embodiments, the method may further comprise a cleavage of the coenzyme A thioester to obtain the free form of the α-branched β'-hydroxy carbonyl compound. Thus, in preferred embodiments the method may further comprise performing a hydrolysis reaction with the coenzyme A thioester of the α-branched β'-hydroxy carbonyl compound under basic conditions or by using a thioesterase.

In a further preferred embodiment, the enzyme-catalyzed reductive aldol reaction may be performed in the presence of an acyl-CoA oxidase. The acyl-CoA oxidase can be used to continually recycle the side product alkyl-CoA back into the α,β-unsaturated carbonyl donor.

### Description of the invention

The present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase.

With other words, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a reductive aldolase in the presence of a cofactor, wherein the reductive aldolase is an enoyl-CoA carboxylase/reductase.

Formulated differently, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a reductive aldolase in the presence of a cofactor, wherein the reductive aldolase is derived from an enoyl-CoA carboxylase/reductase.

The term **"polypeptide capable of catalyzing reductive aldol reactions"** as used herein, refers to an enzyme having the enzymatic activity in which α,β-unsaturated carbonyl donors, such as coenzyme A thioesters of α,β-unsaturated carboxylic acids, are coupled with carbonyl acceptors, such as aldehydes and α-branched β'-hydroxy carbonyl compounds are formed, such as α-branched β'-hydroxy acyl-CoAs as shown below:

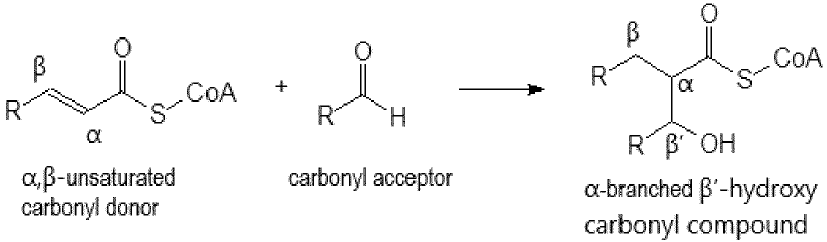

Herein, the "polypeptide capable of catalyzing reductive aldol reactions" is also referred to as **"reductive aldolase".** The newly identified group of enzymes called reductive aldolases are able to couple α,β-unsaturated carbonyl donors with carbonyl acceptors to form α-branched β'-hydroxy carbonyl compounds. Consequently, the term **"reductive aldolase activity"** as used herein refers to the enzymatic activity of catalyzing reductive aldol reactions of α,β-unsaturated carbonyl donors, such as coenzyme A thioesters of α,β-unsaturated carboxylic acids, with carbonyl acceptors, such as aldehydes. According to the method of the present invention, the "polypeptide capable of catalyzing reductive aldol reactions" or the "reductive aldolase" is an enoyl-CoA carboxylase/reductase (Ecr).

The term **"enoyl-CoA carboxylase/reductase"** (Ecr) as used herein refers to a "polypeptide capable of catalyzing reductive aldol reactions" or a "reductive aldolase". Enoyl-CoA carboxylases/reductases (Ecrs) naturally catalyze the reductive carboxylation of enoyl-CoA thioesters. Using NADPH as reducing equivalent, enoyl-CoA carboxylases/reductases (Ecrs) form an enolate intermediate, which is subsequently resolved by CO₂ as electrophile, yielding the corresponding (2S)-alkylmalonyl-CoA. Replacement of the native CO₂ electrophile in enoyl-CoA carboxylase/reductase (Ecr) by different carbonyl acceptors results in the formation of α-branched β'-hydroxy carbonyl compounds, such as α-branched β'-hydroxyacyl-CoAs.

A particular preferred example of an "enoyl-CoA carboxylase/reductase" as used herein refers to **"crotonyl-CoA carboxylase/reductase"** (Ccr). Crotonyl-CoA carboxylase/reductase relates to an enzyme that belongs to EC 1.3.1.85. Crotonyl-CoA carboxylase/reductase naturally catalyzes the reductive carboxylation of crotonyl-CoA to (2S)-ethylmalonyl-CoA by using one equivalent of CO₂ and NAPDH. In absence of CO₂, crotonyl-CoA carboxylase/reductase produces butyryl-CoA. Besides crotonyl-CoA, crotonyl-CoA carboxylase/reductase can also accept acrylyl-CoA. Some homologs have also been shown to carboxylate other enoyl-CoA thioesters containing halogenated or branched acyl side-chains among others. Because these homologs have other substrates than crotonyl-CoA, the entire enzyme family is herein referred to as enoyl-CoA carboxylase/reductase (Ecr).

Thus, the term "enoyl-CoA carboxylase/reductase" (Ecr) as used herein includes naturally occurring or wild type enoyl-CoA carboxylases/reductases such as crotonyl-CoA carboxylase/reductase (Ccr) or also engineered or modified enoyl-CoA carboxylases/reductases such as an engineered or modified crotonyl-CoA carboxylase/reductases that can accept other enoyl-CoA thioesters besides crotonyl-CoA or that can accept a greater number of different aldehydes as carbonyl acceptor. Herein, such modified crotonyl-CoA carboxylases/reductases are particularly preferably derived from a wild type crotonyl-CoA carboxylase/reductase (Ccr). Examples of such modified enoyl-CoA carboxylases/reductases are provided herein.

Moreover, the term "enoyl-CoA carboxylase/reductase" (Ecr) as used herein further includes modified enoyl-CoA carboxylases/reductases (Ecr) that have been modified in such a way that the natural enzymatic-activity to catalyze the carboxylation of enoyl-CoA thioesters is impaired. Thus, "enoyl-CoA carboxylases/reductases" (Ecr) which "lack carboxylase activity", but however are still capable of catalyzing reductive aldol reactions and thus have "reductive aldolase activity" are herein also possible. Examples of such modified enoyl-CoA carboxylases/reductases are provided herein.

Thus, the term "enoyl-CoA carboxylase/reductase" (Ecr) as used herein particularly preferably relates to naturally occurring enoyl-CoA carboxylases/reductases from various bacteria, such as *Kitasatospora setae* or *Caulobacter crescentus* or to polypeptide variants derived from the naturally occurring enoyl-CoA carboxylases/reductases from various bacteria, such as *Kitasatospora setae* or *Caulobacter crescentus.*

**"Percentage of sequence identity"** and "percentage homology" are used interchangeably herein to refer to comparisons among polypeptides, and are determined by comparing two optimally aligned sequences over a comparison window, wherein the portion of the polypeptide sequence in the comparison window may comprise additions or deletions (i.e. gaps) as compared to the reference sequence for optimal alignment of the two sequences. The percentage may be calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Alternatively, the percentage may be calculated by determining the number of positions at which either the identical amino acid residue occurs in both sequences or an amino acid residue is aligned with a gap to yield the number of matched positions, dividing the number of matched positions by the total number of positions in the window of comparison and multiplying the result by 100 to yield the percentage of sequence identity. Those of skill in the art appreciate that there are many established algorithms available to align two sequences.

**"Reference sequence"** refers to a defined sequence used as a basis for a sequence comparison. A reference sequence may be a subset of a larger sequence, for example, a segment of a full-length polypeptide sequence. Since two polypeptides may each (1) comprise a sequence (i.e., a portion of the complete sequence) that is similar between the two sequences, and (2) may further comprise a sequence that is divergent between the two sequences, sequence comparisons between two (or more) polypeptides are typically performed by comparing sequences of the polypeptides over a "comparison window" to identify and compare local regions of sequence similarity. In some embodiments, a "reference sequence" can be based on a primary amino acid sequence, where the reference sequence is a sequence that can have one or more changes in the primary sequence.

**"Substantial identity"** refers to a polypeptide sequence that has at least 80 percent sequence identity, at least 85 percent identity and 89 to 95 percent sequence identity, more usually at least 99 percent sequence identity as compared to a reference sequence over a comparison window. For polypeptides, the term "substantial identity" means that two polypeptide sequences, when optimally aligned, share at least 80 percent sequence identity, preferably at least 89 percent sequence identity, at least 95 percent sequence identity or more (e.g., 99 percent sequence identity). Preferably, residue positions which are not identical differ by conservative amino acid substitutions.

**"Deletion"** refers to modification to the polypeptide by removal of one or more amino acids from the reference polypeptide. Deletions can comprise removal of 1 or more amino acids, 2 or more amino acids, 5 or more amino acids, 10 or more amino acids, 15 or more amino acids, or 20 or more amino acids, up to 10% of the total number of amino acids, or up to 20% of the total number of amino acids making up the reference enzyme while retaining enzymatic activity and/or retaining the improved properties of an engineered enoyl-CoA carboxylase/reductase or engineered reductive aldolase. Deletions can be directed to the internal portions and/or terminal portions of the polypeptide.

**"Insertion"** refers to modification to the polypeptide by addition of one or more amino acids from the reference polypeptide. Insertions can be in the internal portions of the polypeptide, or to the carboxy or amino terminus. Insertions as used herein include fusion proteins as is known in the art. The insertion can be a contiguous segment of amino acids or separated by one or more of the amino acids in the naturally occurring polypeptide.

**"Amino acid substitution"** refers to modification to the polypeptide by substitution of one or more amino acids from the reference polypeptide with other amino acids. Amino acid substitution can comprise substitutions of 1 or more amino acids, 2 or more amino acids, 5 or more amino acids, 10 or more amino acids, 15 or more amino acids, or 20 or more amino acids, up to 10% of the total number of amino acids, or up to 20% of the total number of amino acids making up the reference polypeptide while retaining enzymatic activity of enoyl-CoA carboxylase/reductase or enzymatic activity of reductive aldolase.

**"Isolated polypeptide"** refers to a polypeptide which is substantially separated from other contaminants that naturally accompany it, e.g., protein, lipids, and nucleic acids. The term embraces polypeptides which have been removed or purified from their naturally-occurring environment or expression system (e.g., host cell or in vitro synthesis). The enoyl-CoA carboxylase/reductase may be present within a cell, present in the cellular medium, or prepared in various forms, such as lysates or isolated preparations. As such, in some embodiments, the polypeptide capable of catalyzing reductive aldol reactions in the presence of a cofactor, wherein the polypeptide is an enoyl-CoA carboxylase/reductase, can be an isolated polypeptide.

"**Improved enzyme property**" refers to a polypeptide capable of catalyzing reductive aldol reactions that exhibits an improvement in any enzyme property as compared to a reference enoyl-CoA carboxylase/reductase. For the polypeptides capable of catalyzing reductive aldol reactions described herein, the comparison is generally made to the wild-type enoyl-CoA carboxylase/reductase from which the modified enoyl-CoA carboxylase/reductase is derived, although in some embodiments, the reference polypeptide can be another engineered or modified enoyl-CoA carboxylase/reductase. Enzyme properties for which improvement is desirable include, but are not limited to, enzymatic activity (which can be expressed in terms of percent conversion of the substrate), thermo stability, solvent stability, pH activity profile, cofactor requirements, refractoriness to inhibitors (e.g., substrate or product inhibition), stereospecificity, and stereoselectivity (including enantioselectivity).

"**Increased enzymatic activity**" refers to an improved property of the polypeptides capable of catalyzing reductive aldol reactions, which can be represented by an increase in specific activity (e.g., product produced/time/weight protein) or an increase in percent conversion of the substrate to the product (e.g., percent conversion of starting amount of substrate to product in a specified time period using a specified amount of imine reductase) as compared to the reference enoyl-CoA carboxylase/reductase enzyme. Any property relating to enzyme activity may be affected, including the classical enzyme properties of Kₘ, Vₘₐₓ or k_{cat}, changes of which can lead to increased enzymatic activity. Improvements in enzyme activity can be from about 1.2 times the enzymatic activity of the corresponding wild-type enzyme, to as much as 2 times, 5 times, 10 times, 20 times, 25 times, 50 times or more enzymatic activity than the naturally occurring or another engineered or modified enoyl-CoA carboxylase/reductase from which the enoyl-CoA carboxylase/reductase polypeptides were derived. The activity can be measured by any one of standard assays, such as by monitoring changes in properties of substrates, cofactors, or products. In some embodiments, the amount of products generated can be measured by Liquid Chromatography-Mass Spectrometry (LC-MS). Comparisons of enzyme activities are made using a defined preparation of enzyme, a defined assay under a set condition, and one or more defined substrates. Generally, when lysates are compared, the numbers of cells and the amount of protein assayed are determined as well as use of identical expression systems and identical host cells to minimize variations in amount of enzyme produced by the host cells and present in the lysates.

"**Suitable reaction conditions**" refer to those conditions in the biocatalytic reaction solution (e.g., ranges of enzyme loading, substrate loading, cofactor loading, temperature, pH, buffers, co-solvents, etc.) under which a polypeptide capable of catalyzing reductive aldol reactions is capable of catalyzing the reductive aldol reaction of α,β-unsaturated carbonyl donors, such as coenzyme A thioesters of α,β-unsaturated carboxylic acids, with carbonyl acceptors, such as aldehydes to α-branched β'-hydroxy carbonyl, such as α-branched β'-hydroxy acyl-CoAs. Exemplary "suitable reaction conditions" are provided in the present disclosure and are illustrated by the Examples.

"**Cofactor regeneration system**" or "cofactor recycling system" refers to a set of reactants that participate in a reaction that reduces the oxidized form of the cofactor (e.g., NADP⁺ to NADPH). Cofactors oxidized by the enoyl-CoA carboxylase/reductase catalyzed reductive aldol reaction are regenerated in reduced form by the cofactor regeneration system. Cofactor regeneration systems comprise a stoichiometric reductant that is a source of reducing hydrogen equivalents and is capable of reducing the oxidized form of the cofactor. The cofactor regeneration system may further comprise a catalyst, for example an enzyme catalyst that catalyzes the reduction of the oxidized form of the cofactor by the reductant. Cofactor regeneration systems to regenerate NADH from NAD⁺ or NADPH from NADP⁺, respectively, are known in the art and may be used in the methods described herein.

"**Alkyl**" refers to saturated hydrocarbon groups of from 1 to 10 carbon atoms, either straight chained or branched, more preferably from 1 to 8 carbon atoms, and most preferably 1 to 6 carbon atoms. An alkyl with a specified number of carbon atoms is denoted as C₁-C₈ alkyl and refers to a "**linear C₁-C₈ alkyl**" of -CH₃, -C₂H₅, -C₃H₇, -C₄H₉, -C₅H₁₁, -C₆H₁₃, -C₇H₁₅, -C₈H₁₇, or a "**branched C₁-C₆ alkyl**" including, but not limited to -CH(CH₃)₂, -CH₂-CH(CH₃)₂, -CH(CH₃)-C₂H₅, -C(CH₃)₃, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -C(CH₃)₂-C₃H₇, -CH(CH₃)-CH(CH₃)-C₂H₅, -CH₂-CH(CH₃)-CH(CH₃)₂, -CH₂-C(CH₃)₂-C₂H₅, -CH(CH₃)-C(CH₃)₃, -C(CH₃)₂-CH(CH₃)₂, and -C₂H₄-C(CH₃)₃.

"**Alkenyl**" refers to groups of from 2 to 10 carbon atoms, preferably 2 to 6 carbon atoms (C₂-C₈ alkenyl), either straight or branched containing at least one double bond but optionally containing more than one double bond. As used herein, the term "**linear or branched C₂-C₈ alkenyl**" includes, but is not limited to -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₂H₄-CH=CH₂, -CH=CH-C₂H₅, -CH=C(CH₃)₂, -CH₂-CH=CH-CH₃, -CH=CH-CH=CH₂, C₃H₆-CH=CH₂, -CH=CH-C₃H₇, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, and -CH=CH-Ph.

"**Alkynyl**" refers to groups of from 2 to 10 carbon atoms, preferably from 2 to 8 carbon atoms, either straight or branched containing at least one triple bond but optionally containing more than one triple bond, and additionally optionally containing one or more double bonded moieties. As used herein, the term "**linear or branched C₂-C₈ alkynyl**" includes, but is not limited to -CH₂-C=CH, -C=CH, -C≡C-CH₃, -C₂H₄-C≡CH, -C≡C-C₂H₅, -CH₂-C≡C-CH₃, -C≡C-CH=CH₂, -CH=CH-C=CH, -C=C-C=CH, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₄H₃-C≡CH, -C≡C-C₄H₉, -C₃H₆-C≡C-CH₃, -CH₂-C≡C-C₃H₇, and -C=C-Ph.

**"Cycloalkyl"** refers to cyclic alkyl groups of from 3 to 12 carbon atoms, preferably from 3 to 8 carbon atoms, having a single cyclic ring or multiple condensed rings which can be optionally substituted with from 1 to 3 alkyl groups. Exemplary cycloalkyl groups include, but are not limited to, single ring structures such as cyclopropyl, cyclobutyl, cyclopentyl, cyclooctyl, 1-methylcyclopropyl, 2-methylcyclopentyl, 2-methylcyclooctyl, and the like, or multiple ring structures, including bridged ring systems, such as adamantyl. As used herein, **"C₃-C₈ cycloalkyl**" refers to cyclo-C₃H₅, cyclo-C₄H₇, cyclo-C₅H₉, cyclo-C₆H₁₁, cyclo-C₇H₁₃, and cyclo-C₈H₁₅.

"**Aryl**" refers to an unsaturated aromatic carbocyclic group of from 6 to 12 carbon atoms inclusively having a single ring (*e.g.*, phenyl) or multiple condensed rings (*e.g.*, naphthyl or anthryl). Exemplary aryls include phenyl, pyridyl, naphthyl and the like. "**Arylalkyl**" refers to an alkyl substituted with an aryl, *i.e.*, aryl-alkyl groups, preferably having from 1 to 6 carbon atoms in the alkyl moiety and from 6 to 12 carbon atoms inclusively in the aryl moiety. Such arylalkyl groups are exemplified by benzyl, phenethyl and the like. As used herein, the term **"C₆-C₈ arylalkyl**" includes, but is not limited to -CH₂-Ph and -C₂H₄Ph.

"**Heteroalkyl**, "**heteroalkenyl**," and "**heteroalkynyl**," refer to alkyl, alkenyl and alkynyl as defined herein in which one or more of the carbon atoms are each independently replaced with the same or different heteroatoms or heteroatomic groups. Heteroatoms and/or heteroatomic groups which can replace the carbon atoms include, but are not limited to, -O-, -S-, -S-O-, -NR^{α}-, -PH-, -S(O)-, -S(O)₂-,-S(O)NR^{α}-, -S(O)₂NR^{α}-, and the like, including combinations thereof, where each R^{α} is independently selected from hydrogen, alkyl, heteroalkyl, cycloalkyl, heterocycloalkyl, aryl, and heteroaryl. "**Heteroaryl**" refers to an aromatic heterocyclic group of from 1 to 10 carbon atoms and 1 to 4 heteroatoms selected from oxygen, nitrogen and sulfur within the ring. Such heteroaryl groups can have a single ring (*e.g.*, pyridyl or furyl) or multiple condensed rings (*e.g.*, indolizinyl or benzothienyl). As used herein, the term "C₁-C₁₀ heteroaryl" refers to aromatic residues with one or more heteroatoms such as O, S, N. "**Heteroarylalkyl**" refers to an alkyl substituted with a heteroaryl, *i.e.*, heteroaryl-alkyl-groups, preferably having from 1 to 6 carbon atoms in the alkyl moiety and from 5 to 12 ring atoms inclusively in the heteroaryl moiety. Such heteroarylalkyl groups are exemplified by pyridylmethyl and the like. "**Heterocycloalkyl**" refers to a saturated or unsaturated group having a single ring or multiple condensed rings, from 2 to 9 carbon ring atoms and from 1 to 4 hetero ring atoms inclusively selected from nitrogen, sulfur or oxygen within the ring. Such heterocyclic groups can have a single ring (*e.g.*, piperidinyl or tetrahydrofuryl) or multiple condensed rings (*e.g.*, indolinyl, dihydrobenzofuran or quinuclidinyl). Examples of heterocycles include, but are not limited to, furan, thiophene, thiazole, oxazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, pyrrolidine, indoline and the like.

"**Oxy**" refers to a divalent group -O-, which may have various substituents to form different oxy groups, including ethers and esters. **"Alkoxy"** or **"alkyloxy"** are used interchangeably herein to refer to the group -OR^{α}, wherein R^{α} is an alkyl group, including optionally substituted alkyl groups. **"Aryloxy"** as used herein refer to the group -OR^{α} wherein R^{α} is an aryl group as defined above including optionally substituted aryl groups as also defined herein.

**"Carboxy"** refers to -COOH. **"Carboxyalkyl"** refers to an alkyl substituted with a carboxy group.

**"Carbonyl"** refers to the group -C(O)-. Substituted carbonyl refers to the group R^{α}-C(O)-R^{α}, where each R^{α} is independently selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical substituted carbonyl groups including acids, ketones, aldehydes, amides, esters, acyl halides, thioesters, and the like.

**"Amino"** refers to the group -NH₂. Substituted amino refers to the group -NHR^{α}, NR^{α}R^{α}, and NR^{α}R^{α}R^{α}, where each R^{α} is independently selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical amino groups include, but are limited to, dimethylamino, diethylamino, trimethylammonium, triethylammonium, methylysulfonylamino, furanyl-oxy-sulfamino, and the like. **"Aminoalkyl"** refers to an alkyl group in which one or more of the hydrogen atoms are replaced with an amino group, including a substituted amino group. **"Aminocarbonyl"** refers to a carbonyl group substituted with an amino group, including a substituted amino group, as defined herein, and includes amides. **"Aminocarbonylalkyl"** refers to an alkyl substituted with an aminocarbonyl group, as defined herein.

**"Halogen"** or "halo" refers to fluoro, chloro, bromo and iodo. **"Haloalkyl"** refers to an alkyl group in which one or more of the hydrogen atoms are replaced with a halogen. Thus, the term "haloalkyl" is meant to include monohaloalkyls, dihaloalkyls, trihaloalkyls, etc. up to perhaloalkyls. As used herein, the expression "C₁-C₂ haloalkyl" includes 1-fluoromethyl, difluoromethyl, trifluoromethyl, 1-fluoroethyl, 1,1-difluoroethyl, 1,2-difluoroethyl, 1,1,1 trifluoroethyl, perfluoroethyl, etc.

**"Hydroxy"** refers to -OH. **"Hydroxyalkyl"** refers to an alkyl substituted with one or more hydroxy group.

**"Thio"** or "sulfanyl" refers to -SH. Substituted thio or sulfanyl refers to -S-R^{α}, where R^{α} is an alkyl, aryl or other suitable substituent. **"Alkylthio"** refers to -SR^{α}, where R^{α} is an alkyl, which can be optionally substituted. Typical alkylthio group include, but are not limited to, methylthio, ethylthio, n-propylthio, and the like. **"Alkylthioalkyl"** refers to an alkyl substituted with an alkylthio group, -SR^{α}, where R^{α} is an alkyl, which can be optionally substituted. **"Thiocarbonyl"** refers to a carbonyl group substituted with a thio group, including a substituted thio group, as defined herein, and includes thioesters.

**"Optionally substituted"** as used herein with respect to the foregoing chemical groups means that positions of the chemical group occupied by hydrogen can be substituted with another atom, such as carbon, oxygen, nitrogen, or sulfur, or a chemical group, exemplified by, but not limited to, hydroxy, oxo, nitro, methoxy, ethoxy, alkoxy, substituted alkoxy, trifluoromethoxy, haloalkoxy, fluoro, chloro, bromo, iodo, halo, methyl, ethyl, propyl, butyl, alkyl, alkenyl, alkynyl, substituted alkyl, trifluoromethyl, haloalkyl, hydroxyalkyl, alkoxyalkyl, thio, alkylthio, acyl, carboxy, alkoxycarbonyl, carboxamido, substituted carboxamido, alkylsulfonyl, alkylsulfinyl, alkylsulfonylamino, sulfonamido, substituted sulfonamido, cyano, amino, substituted amino, alkylamino, dialkylamino, aminoalkyl, acylamino, amidino, amidoximo, hydroxamoyl, phenyl, aryl, substituted aryl, aryloxy, arylalkyl, arylalkenyl, arylalkynyl, pyridyl, imidazolyl, heteroaryl, substituted heteroaryl, heteroaryloxy, heteroarylalkyl, heteroarylalkenyl, heteroarylalkynyl, cyclopropyl, cyclobutyl, cyclopentyl, cyclohexyl, cycloalkyl, cycloalkenyl, cycloalkylalkyl, substituted cycloalkyl, cycloalkyloxy, pyrrolidinyl, piperidinyl, morpholino, heterocycle, (heterocycle)oxy, and (heterocyclyl)alkyl; where preferred heteroatoms are oxygen, nitrogen, and sulfur. However, it is clear to a skilled person that the term "can be substituted" refers to the replacement of a hydrogen atom by one of the above-mentioned chemical groups. One of ordinary skill in the art would understand that with respect to any chemical group described as optionally substituted, only sterically practical and/or synthetically feasible chemical groups are meant to be included.

The present invention relates to a method for preparing α-branched β'-hydroxy carbonyl compounds by enzymatic-catalyzed reductive aldol reaction including the use of enoyl-CoA carboxylases/reductases (Ecrs) as a polypeptides capable of catalyzing reductive aldol reactions. The inventors of the present invention have found that the active site of enoyl-CoA carboxylases/reductases (Ecrs) can be exploited for reductive aldol reactions. Thus, the present invention provides the first enzyme-based approach to this highly relevant C-C bond forming reaction, belonging to the standard repertoire of organic synthesis. This new-to-nature reaction is enabled by naturally existing dipolar moments in the enoyl-CoA carboxylase/reductase (Ecr) scaffold that allow carbonyl acceptors such as formaldehyde and other aldehydes to align for the reaction at the active site, even in the absence of key residues that are otherwise essential for the native carboxylation reaction of enoyl-CoA carboxylases/reductases (Ecrs).

Beyond organic synthesis, formaldehyde condensation reactions have recently gained interest for establishing a versatile one-carbon bioeconomy. Compared to other formaldehyde condensing enzymes, enoyl-CoA carboxylase/reductase (Ecr) surpasses the kinetic parameters of recently engineered enzymes by several orders of magnitude and shows comparable activities to natural existing enzymes, such as 3-hexulose-6-phosphate synthase, which opens the way for the use of enoyl-CoA carboxylase/reductase (Ecr) for the design and realization of synthetic C1-assimilation pathways. Concluding, reductive aldol C-C bond formations represent a new-to-nature reaction that expand the portfolio of naturally existing enzymatic transformations for the synthesis of valuable α-branched-β'-hydroxyacyl-CoA building blocks of increasing interest in organic synthesis, biocatalysis and synthetic biology.

Thus, the inventors of the present invention sought to develop a "reductive aldolase" by exploiting the catalytic site of enoyl-CoA carboxylases/reductases (Ecrs).

It has been surprisingly found that crotonyl-CoA carboxylase/reductase (Ccr) from *Kitasatospora setae* (KsCcr) is able to natively react with formaldehyde as an alternative electrophile to form α-branched 3-hydroxyacyl-thioester at high turnover frequency, catalytic efficiency and exquisite stereoselectivity. Furthermore, it has been found surprisingly found that KsCcr also accepts acetaldehyde, enabling the generation of 3-hydroxyacyl-thioesters with two stereocenters from their corresponding enoyl-CoA substrates at high yield and diastereomeric ratio.

KsCcr wild-type (WT) natively promotes the NADPH-dependent reductive carboxylation of crotonyl-CoA into ethylmalonyl-CoA. To determine if KsCcr was able to catalyze a reductive aldol reaction the enzyme was incubated with 1 mM NADPH, 500 µM crotonyl-CoA, and 75 mM formaldehyde (instead of the native electrophile CO₂). Under these conditions, neither ethylmalonyl-CoA (carboxylation product) nor butyryl-CoA (reduction side product) were detected with LC-MS (see Table 2 further below). However, LC-MS analysis showed formation of a compound with the expected mass and an expected fragmentation pattern corresponding to putative aldol product 2-(hydroxymethyl)-butyryl-CoA (2-HMB-CoA). The compound was isolated and its structure was further verified using 2-dimensional NMR and high-resolution MS experiments. Proton correlation spectra (COSY) showed a correlation between the C_{α}-proton of the acyl-CoA ester and a newly introduced proton at C_{β}, indicative of a newly formed C-C bond. The newly introduced carbon was also observed in the ¹³C-NMR experiment, confirming formation of aldol product 2-HMB-CoA by KsCcr.

KsCcr WT showed an apparent turnover rate of 30 ± 1 s⁻¹ and apparent K_{M} values of 33 ± 4 µM and 389 ± 49 µM for crotonyl-CoA and NADPH, respectively (see Table 1 further below). The apparent K_{M} value for formaldehyde (32 ± 8 mM) corresponds to a K_{M} 32 ± 8 µM of active carbonyl form of formaldehyde under the chosen reaction conditions. Under sub-saturating formaldehyde concentrations, product distribution was shifted from aldol product 2-HMB-CoA towards the reduction product butyryl-CoA, indicating that the enolate intermediate was protonated instead of reacting with formaldehyde. This behavior reminds of the native carboxylation reaction of KsCcr, when the concentration of the natural electrophile CO₂ is lowered below K_{M}, which also results in increased side product formation. Overall, these results confirmed that formaldehyde serves as substrate for KsCcr WT

The reaction catalyzed by KsCcr generates an (S)-configured stereocenter at the α-position of the reaction product. When using higher substituted aldehydes, a second stereocenter would be generated at the β-position, which would be highly interesting for synthetic organic applications. It was first tested, whether KsCcr would accept longer chain aldehydes. Indeed, in the presence of 500 mM acetaldehyde, the enzyme formed 35% of 2-ethyl-3-hydroxybutyryl-CoA (2-EHB-CoA) over total products, with substantial amounts of butyryl-CoA as a side product. To improve yield, an acyl-CoA oxidase was used to continually recycle the side product butyryl-CoA back into the substrate crotonyl-CoA. With this proof-reading strategy the yield of the reaction could be increased up to 72%. High resolution mass spectrometry together with ¹H-¹H COSY and ¹³C-NMR experiments confirmed the structure of 2-EHB-CoA.

To determine the stereochemistry at the β-position the reaction was analyzed by LC-MS and compared the retention times to chemically synthesized (2S,3R)-EHB-CoA and (2*S*,3*S*)-EHB-CoA standards. These experiments showed that KsCcr WT produces a mixture of (2*S*,3*R*)-EHB-CoA and (2*S*,3*S*)-EHB-CoA at a diastereomeric ratio (*dr*) of 82:17 for the (2*S*,3*R*)-diastereomer (see Table 3 further below), demonstrating that KsCcr natively carries the potential to for stereospecific catalysis of reductive aldol reactions.

The KsCcr WT was also tested with propionaldehyde and the propionaldehyde product could be detected by MS. However, the conversion was strongly decreased in comparison to the conversion of formaldehyde or acetaldehyde, respectively. Thus, in embodiments, wherein the polypeptide is a wild type crotonyl-CoA carboxylase/reductase it is particularly preferred that the carbonyl acceptor is formaldehyde or acetaldehyde.

Thus, a preferred embodiment of the present invention relates in particular to a method for preparing an α-branched β'-hydroxy carbonyl compound by reductive aldol reaction, wherein the method comprises the steps:
a) providing an α,β-unsaturated carbonyl donor, wherein the α,β-unsaturated carbonyl donor is crotonyl-CoA;
b) providing a carbonyl acceptor, wherein the carbonyl acceptor is an aldehyde selected from formaldehyde or acetaldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of NADPH, wherein the polypeptide is a wild type crotonyl-CoA carboxylase/reductase.

In preferred embodiments, the enzyme-catalyzed reductive aldol reaction is performed in the presence of an acyl-CoA oxidase.

In further embodiments, the enzymatic-catalyzed reductive aldol reaction is performed in the absence of CO₂. However, CO₂ does not have to be completely avoided and running the reactions at ambient conditions is possible. The avoidance of CO₂ does not have to be very strict as even under ambient conditions KsCcr WT is able to generate >95% of aldol product.

Preferably, the polypeptide capable of catalyzing reductive aldol reactions may be a wild type crotonyl-CoA carboxylase/reductase obtained from *Kitasatospora setae.* With other words, the polypeptide capable of catalyzing reductive aldol reactions may be a wild type crotonyl-CoA carboxylase/reductase obtained from *Kitasatospora setae* comprising the amino acid sequence of SEQ ID NO: 1.

Preferably, the polypeptide capable of catalyzing reductive aldol reactions may be a wild type crotonyl-CoA carboxylase/reductase obtained from *Kitasatospora setae* comprising the amino acid sequence of SEQ ID NO: 1:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSHFANYREAFEANRLV
AKGKIHPTLSKVYALEETGQAALDVHHNKHQGKVGVLCLAPREGLGVTDPELRS
KHLTKINAFRNV.

In further preferred embodiments, the polypeptide capable of catalyzing reductive aldol reactions may be also a wild type crotonyl-CoA carboxylase/reductase obtained also from other microorganism besides *Kitasatospora setae.*

First empirical data have shown that besides crotonyl-CoA carboxylase/reductase (KsCcr), also crotonyl-CoA carboxylase/reductase obtained from *Caulobacter crescentus* (CcCcr) is able to catalyze a reductive aldol reaction.

Thus, in further preferred embodiments, the polypeptide capable of catalyzing reductive aldol reactions may be a wild type crotonyl-CoA carboxylase/reductase obtained *Caulobacter crescentus.* With other words, the polypeptide capable of catalyzing reductive aldol reactions may be a wild type crotonyl-CoA carboxylase/reductase obtained *Caulobacter crescentus* comprising the amino acid sequence of SEQ ID NO: 2.

Preferably, the polypeptide capable of catalyzing reductive aldol reactions may be a wild type crotonyl-CoA carboxylase/reductase obtained *Caulobacter crescentus* comprising the amino acid sequence of SEQ ID NO: 2:

MTIQTLETTALKDLYEIGEIPPAFHVPKTMYAWSIRKERHGKPTQAMQVEVVPTW
EIGEDEVLVLVMAAGVNYNGVWAALGEPISPLDGHKQPFHIAGSDASGIVWKVG
AKVKRWKLGDEVVIHCNQDDGDDEECNGGDPMFSSSQRIWGYETPDGSFAQF
CRVQSRQLLPRPKHLTWEESACYTLTLATAYRMLFGHKPHELKPGQNVLVWGA
SGGLGVFATQLAAVAGANAIGVVSSEDKREFVLSMGAKAVLNRGEFNCWGQLP
KVNGPEFNDYMKESRKFGKAIWQITGNKDVDMVFEHPGEQTFPVSVFLVKRGG
MVVICAGTTGFNLTMDARFLWMRQKRVQGSHFANLMQASAANQLVIDRRVDPC
LSEVFPWDQIPAAHEKMLANQHLPGNMAVLVCAQRPGLRTFEEVQELSGAP.

In further preferred embodiments the polypeptide capable of catalyzing reductive aldol reactions may be a wild type crotonyl-CoA carboxylase/reductase comprising at least 95% sequence identity to amino acid sequence SEQ ID NO: 1 or SEQ ID NO:2. Still more preferably, the polypeptide capable of catalyzing reductive aldol reactions may be a wild type crotonyl-CoA carboxylase/reductase comprising at least 95% sequence identity to amino acid sequence SEQ ID NO: 1.

Given the similarity of the active structure, also other wild type crotonyl-CoA carboxylases/reductases from other bacteria may be used in the method of the present invention. Thus, further wild type crotonyl-CoA carboxylases/reductases, which may be used in the method of the present invention, may be found in many bacteria such as *Rhodobacter sphaeroides (Cereibacter sphaeroides),* actinobacteria including, but not limited to *Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis,* betaproteobacteria including, but not limited to *Burkholderia ambifaria, campylobacteria* including, but not limited to *Arcobacter nitrofigilis DSM 7299,* deltaproteobacteria including, but not limited to *Desulfovibrio desulfuricans,* Firmicutes including, but not limited to *Ruminococcus champanellensis,* Gammaproteobacteria including, but not limited *to Pectobacterium atrosepticum, Pseudomonas syringae,* spirochaetota including, but not limited to *Leptospira borgpetersenii, Leptospira santarosai, Leptospira yanagawae.*

Thus, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the enoyl-CoA carboxylase/reductase is obtained from *Kitasatospora setae, Caulobacter crescentus, Rhodobacter sphaeroides (Cereibacter sphaeroides), Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis, Burkholderia ambifaria, Arcobacter nitrofigilis DSM* 7299, *Desulfovibrio desulfuricans, Ruminococcus champanellensis, Pectobacterium atrosepticum, Pseudomonas syringae, Leptospira borgpetersenii, Leptospira santarosai or Leptospira yanagawae.*

Thus, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase obtained from *Kitasatospora setae, Caulobacter crescentus, Rhodobacter sphaeroides (Cereibacter sphaeroides), Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis, Burkholderia ambifaria, Arcobacter nitrofigilis DSM* 7299, *Desulfovibrio desulfuricans, Ruminococcus champanellensis, Pectobacterium atrosepticum, Pseudomonas syringae, Leptospira borgpetersenii, Leptospira santarosai or Leptospira yanagawae.*

Thus, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a wild type crotonyl-CoA carboxylase/reductase obtained from *Kitasatospora setae, Caulobacter crescentus, Rhodobacter sphaeroides (Cereibacter sphaeroides), Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis, Burkholderia ambifaria, Arcobacter nitrofigilis DSM 7299*, *Desulfovibrio desulfuricans, Ruminococcus champanellensis, Pectobacterium atrosepticum, Pseudomonas syringae, Leptospira borgpetersenii, Leptospira santarosai or Leptospira yanagawae.*

Thus, the present invention relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde; and
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a modified crotonyl-CoA carboxylase/reductase obtained from *Kitasatospora setae, Caulobacter crescentus, Rhodobacter sphaeroides (Cereibacter sphaeroides), Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis, Burkholderia ambifaria, Arcobacter nitrofigilis DSM 7299*, *Desulfovibrio desulfuricans, Ruminococcus champanellensis, Pectobacterium atrosepticum, Pseudomonas syringae, Leptospira borgpetersenii, Leptospira santarosai or Leptospira yanagawae.*

Preferably the enoyl-CoA carboxylase/reductase is obtained from *Kitasatospora setae* or *Caulobacter crescentus.* The inventors of the present invention have identified several conserved parts of the protein sequence between KsCcr and CcCcr, which are given below (in parentheses is the amino acid numbering corresponding to the respective wildtype numbering in SEQ ID NO: 1 or SEQ ID NO: 2):

**Table 1: Conserved parts of the protein sequence between KsCcr and CcCcr**

| SEQ ID | Corresponding amino acid numbering in SEQ ID NO: 1 or SEQ ID NO: 2 | conserved parts of the protein sequence |
|---|---|---|
| SEQ ID NO: 3 | SEQ ID NO: 1 (68-84) | EALVAVMASSVNYNTVW |
| SEQ ID NO: 4 | SEQ ID NO: 2 (61-77) | EVLVLVMAAGVNYNGVW |
| SEQ ID NO: 5 | SEQ ID NO: 1 (113-145) | PYHVLGSDLAGVVLRTGAGVN AWKPGDEVVAHC |
| SEQ ID NO: 6 | SEQ ID NO: 2 (93-125) | PFHIAGSDASGIVWKVGAKVK RWKLGDEVVIHC |
| SEQ ID NO: 7 | SEQ ID NO: 1 (165-172) | QRIWGFET |
| SEQ ID NO: 8 | SEQ ID NO: 2 (146-153) | QRIWGYET |
| SEQ ID NO: 9 | SEQ ID NO: 1 (187-201) | QLLPKPKHLTWEEAA |
| SEQ ID NO: 10 | SEQ ID NO: 2 (168-182) | QLLPRPKHLTWEESA |
| SEQ ID NO: 11 | SEQ ID NO: 1 (208-214) | TAYRQL |
| SEQ ID NO: 12 | SEQ ID NO: 2 (190-195) | TAYRML |
| SEQ ID NO: 13 | SEQ ID NO: 1 (227-258) | NVLIWGASGGLGSYATQYALA GGATPICVVSS |
| SEQ ID NO: 14 | SEQ ID NO: 2 (208-239) | NVLVWGASGGLGVFATQLAA VAGANAIGVVSS |
| SEQ ID NO: 15 | SEQ ID NO: 1 (310-319) | DVDIVFEHPG |
| SEQ ID NO: 16 | SEQ ID NO: 2 (296-305) | DVDMVFEHPG |
| SEQ ID NO: 17 | SEQ ID NO: 1 (363-368) | GSHFAN |
| SEQ ID NO: 18 | SEQ ID NO: 2 (347-354) | GSHFAN |

Residues N81 (present in SEQ ID NO 3), F170 (present in SEQ ID NO 7), E171 (present in SEQ ID NO 7), and H365 (present in SEQ ID NO 17), (Ks numbering) have been shown to be highly important for the wildtype carboxylation activity. For increasing the reductive aldol reaction, these residues were also mutated by amino acid substitution. T82 (present in SEQ ID NO 3) and S119 (present in SEQ ID NO 5) are responsible for orienting N81 which is important for the native carboxylation reactivity. However, the new-to-nature reductive aldol reactivity is much less affected by a mutation of these residues.

SEQ ID NO: 3 contains the important active site residues N81. SEQ ID NO: 5 contains mostly the core of the enzyme but also residue Ser119 responsible for orienting N81. SEQ ID NO: 7: these residues interact with the CoA ester and are therefore important for binding of the CoA ester substrate. SEQ ID NO: 9 relates to a conserved sequence but these residues do not interact the substrates and therefore amino acid substation in the other conserved sequences are more preferred. SEQ ID NO: 11: these residues form a helix which lies close to the NADPH binding site. SEQ ID NO: 13: these residues form two parallel beta sheets and a helix which lie close to the NADPH binding site. SEQ ID NO: 15: these residues form a beta sheet which lies close to the NADPH binding site. SEQ ID NO: 17: these residues lie on a flexible loop containing the important active site residue H365.

In further preferred embodiments the polypeptide capable of catalyzing reductive aldol reactions may be a wild type crotonyl-CoA carboxylase/reductase comprising conserved parts having at least 80% sequence identity of conserved parts of peptide sequences as set forth in SEQ ID NO: 3, SEQ ID NO: 5, SEQ ID NO: 7, SEQ ID NO: 9, SEQ ID NO: 11, SEQ ID NO: 13, SEQ ID NO: 15 and SEQ ID NO: 17.

In more preferred embodiments the polypeptide capable of catalyzing reductive aldol reactions may be a wild type crotonyl-CoA carboxylase/reductase comprising conserved parts having at least 80% sequence identity of conserved parts of peptide sequences as set forth in SEQ ID NO: 3, SEQ ID NO: 5, SEQ ID NO: 7 and SEQ ID NO: 17.

In further preferred embodiments the polypeptide capable of catalyzing reductive aldol reactions may be a modified crotonyl-CoA carboxylase/reductase comprising one or more amino acid substitutions in conserved parts having at least 80% sequence identity of conserved parts of peptide sequences as set forth in SEQ ID NO: 3, SEQ ID NO: 5, SEQ ID NO: 7, SEQ ID NO: 9, SEQ ID NO: 11, SEQ ID NO: 13, SEQ ID NO: 15 and SEQ ID NO: 17.

In more preferred embodiments the polypeptide capable of catalyzing reductive aldol reactions may be a wild type crotonyl-CoA carboxylase/reductase comprising one or more amino acid substitutions in conserved parts having at least 80% sequence identity of conserved parts of peptide sequences as set forth in SEQ ID NO: 3, SEQ ID NO: 5, SEQ ID NO: 7 and SEQ ID NO: 17.

In further preferred embodiments the polypeptide capable of catalyzing reductive aldol reactions may be a wild type crotonyl-CoA carboxylase/reductase comprising conserved parts having at least 80% sequence identity of conserved parts of peptide sequences as set forth in SEQ ID NO: 4, SEQ ID NO: 6, SEQ ID NO: 8, SEQ ID NO: 10, SEQ ID NO: 12, SEQ ID NO: 14, SEQ ID NO: 16 and SEQ ID NO: 18.

In more preferred embodiments the polypeptide capable of catalyzing reductive aldol reactions may be a wild type crotonyl-CoA carboxylase/reductase comprising conserved parts having at least 80% sequence identity of conserved parts of peptide sequences as set forth in SEQ ID NO: 4, SEQ ID NO: 6, SEQ ID NO: 8, and SEQ ID NO: 18.

In further preferred embodiments the polypeptide capable of catalyzing reductive aldol reactions may be a wild type crotonyl-CoA carboxylase/reductase comprising one or more amino acid substitutions in conserved parts having at least 80% sequence identity of conserved parts of peptide sequences as set forth in SEQ ID NO: 4, SEQ ID NO: 6, SEQ ID NO: 8, SEQ ID NO: 10, SEQ ID NO: 12, SEQ ID NO: 14, SEQ ID NO: 16 and SEQ ID NO: 18.

In more preferred embodiments the polypeptide capable of catalyzing reductive aldol reactions may be a wild type crotonyl-CoA carboxylase/reductase comprising one or more amino acid substitutions in conserved parts having at least 80% sequence identity of conserved parts of peptide sequences as set forth in SEQ ID NO: 4, SEQ ID NO: 6, SEQ ID NO: 8, and SEQ ID NO: 18.

It has been found that other wild type crotonyl-CoA carboxylases/reductases from other bacteria such as *Rhodobacter sphaeroides (Cereibacter sphaeroides),* actinobacteria including, but not limited to *Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis,* betaproteobacteria including, but not limited to *Burkholderia ambifaria, campylobacteria* including, but not limited to *Arcobacter nitrofigilis DSM 7299,* deltaproteobacteria including, but not limited to *Desulfovibrio desulfuricans,* Firmicutes including, but not limited to *Ruminococcus champanellensis,* Gammaproteobacteria including, but not limited to *Pectobacterium atrosepticum, Pseudomonas syringae,* spirochaetota including, but not limited to *Leptospira borgpetersenii, Leptospira santarosai, Leptospira yanagawae* comprise conserved parts having at least 80% sequence identity of conserved parts of peptide sequences as set forth in SEQ ID NO: 3, SEQ ID NO: 5, SEQ ID NO: 7, and SEQ ID NO: 17 or comprising conserved parts having at least 80% sequence identity of conserved parts of peptide sequences as set forth in SEQ ID NO: 4, SEQ ID NO: 6, SEQ ID NO: 8, and SEQ ID NO: 18.

Thus, also modified crotonyl-CoA carboxylases/reductases obtained from other bacteria such as *Rhodobacter sphaeroides (Cereibacter sphaeroides),* actinobacteria including, but not limited to *Streptomyces varsoviensis, Actinoplanes sp. N902-109, Actinosynnema mirum,* Actinosynnema mirum, Catenulispora acidiphila, *Conexibacter woesei, Conexibacter woesei, Conexibacter woesei, Frankia sp. Eul1c, Frankia sp. QA3, Geodermatophilus obscurus, Gordonia effuse, Ilumatobacter coccineum, Microthrix parvicella, Mobilicoccus pelagius, Nocardiopsis alba, Nocardiopsis dassonvillei, Pseudonocardia dioxanivorans, Rhodococcus fascians, Rhodococcus opacus, Saccharomonospora xinjiangensis, Saccharothrix espanaensis, Salinispora tropica CNB-440, Stackebrandtia nassauensis, Streptomyces albus, Streptomyces auratus, Streptomyces avermitilis, Streptomyces bingchenggensis, Streptomyces bingchenggensis BCW-1, Streptomyces bottropensis 2, Streptomyces cattleya, Streptomyces coelicolor A3, Streptomyces collinus, Streptomyces davawensis, Streptomyces filamentosus, Streptomyces ipomoeae, Streptomyces lavendulae, Streptomyces mobaraensis, Streptomyces nanchangensis, Streptomyces pristinaespiralis, Streptomyces sp. C, Streptomyces sp. CNH-287, Streptomyces sp. HKI0576 (DivD), Streptomyces sp. HKI0576 (DivR), Streptomyces sp. Mg1, Streptomyces sp. SirexAA-E, Streptomyces sp. W007, Streptomyces sviceus, Streptomyces tsukubaensis, Streptomyces viridochromogenes, Streptosporangium roseum, Thermomonospora curvata, Tsukamurella paurometabola,* alphaproteobacteria including, but not limited to *Azorhizobium caulinodans, Hyphomicrobium denitrificans, Labrenzia aggregata, Loktanella vestfoldensis, Magnetospirillum sp. SO-1, Maritimibacter alkaliphilus, Mesorhizobium metallidurans, Methylobacterium radiotolerans, Pelagibaca bermudensis, Phaeobacter inhibens, Thalassospira xiamenensis,* betaproteobacteria including, but not limited to *Burkholderia ambifaria, campylobacteria* including, but not limited to *Arcobacter nitrofigilis DSM 7299,* deltaproteobacteria including, but not limited to *Desulfovibrio desulfuricans,* Firmicutes including, but not limited to *Ruminococcus champanellensis,* Gammaproteobacteria including, but not limited *to Pectobacterium atrosepticum, Pseudomonas syringae,* spirochaetota including, but not limited to *Leptospira borgpetersenii, Leptospira santarosai, Leptospira yanagawae,* are herein preferred which comprise one or more amino acid substitutions in conserved parts having at least 80% sequence identity of conserved parts of peptide sequences as set forth in SEQ ID NO: 3, SEQ ID NO: 5, SEQ ID NO: 7, and SEQ ID NO: 17 or in conserved parts having at least 80% sequence identity of conserved parts of peptide sequences as set forth in SEQ ID NO: 4, SEQ ID NO: 6, SEQ ID NO: 8, and SEQ ID NO: 18.

In further embodiments of the present invention enzyme engineering was used to design active site variants with altered diastereoselectivity and extended catalytic scope to access 16 different compounds in a proof-of-principle. Overall, the present invention sets the theoretical and experimental foundation for the engineering of reductive aldolases, a new-to-nature enzymatic activity of great potential for bio- and organo-catalysis.

For the native reductive carboxylation reaction of KsCcr WT, it was previously established that the reaction with the CO₂ electrophile involves four key residues, Asn81, Phe170, Glu171, and His365, as well as an ordered water molecule, coordinated by Glu171 and His365. In contrast to CO₂, however, formaldehyde has a significant dipole moment, which raised the question of how the formaldehyde molecule would interact with active site residues during the reaction. Free energy calculations of the ternary complex of crotonyl-CoA and NADPH with KsCcr indicated that His365 has an increased pKa value in the active site of the reactive closed subunits of KsCcr WT (ΔpKₐ = 2.3±0.3). In the calculations, the protonated sidechain of His365 appears hydrogen-bonded to the ordered water molecule that bridges to the negatively charged sidechain of Glu171, which creates a net dipole moment in the cavity and aligns formaldehyde in the reactant state (Figure 2A).

To establish the enzyme's reaction mechanism, the minimum free energy path (MFEP) was calculated with QM/MM molecular dynamics simulations starting from crotonyl-CoA, NADPH and formaldehyde via the enolate to 2-HMB-CoA and NADP⁺ (Figure 2A). During the reaction, formaldehyde approaches crotonyl-CoA and establishes a hydrogen bond interaction between its carbonyl oxygen atom and the ordered water molecule by the rotation of both molecules. This reorientation follows a hydride transfer from NADPH to the β-position of crotonyl-CoA at the transition state (Figure 2B) with a free energy barrier at the DFTB3/CHARMM36 level of 12.3 ± 0.5 kcal/mol, which is in reasonable agreement with the value calculated from the observed catalytic rate constant (15.5 kcal/mol). After transition state, the nucleophilic enolate attacks formaldehyde in a barrierless process giving rise to a reactive alkoxy species (Figure 2C). This reactive species is subsequently protonated by the ordered water molecule to form 2-HMB-CoA, followed by a proton transfer from His365 (Figure 2D), yielding the 2-HMB-CoA product and deprotonated His365 at the end of the reaction (Figure 2E). His365 is likely re-protonated during the conformational change between the open and closed state of both active sites in the dimer associated with crotonyl-CoA binding.

To verify the importance of His365, a steady-state analysis of the KsCcr variant His365Asn was performed, which showed that the enzyme is severely affected in catalysis. The apparent catalytic rate decreased to 10 % of the WT enzyme (k_{cat} = 3.1 ± 0.2 s⁻¹, Table 2), while the apparent K_{M} for formaldehyde was increased twofold compared to WT (K_{M} = 66 ± 23 µM). However, at 75mM formaldehyde, the enzyme still produced 89 % of 2-HMB-CoA, indicating the presence of an alternative proton donor. Free energy calculations suggested that in the His365Asn variant, the pKa value of Glu171 increases by 2 pKa units. This renders Glu171 partially protonated in the His365Asn variant, so that the residue is able to donate a proton to the alkoxy intermediate, albeit at reduced rate under the chosen experimental conditions. Overall, these results established the roles of His365 and Glu171 in substrate binding and the final protonation step of the reaction, identifying essential differences in the interaction of the enzyme with its native substrate CO₂ and its non-native substrate formaldehyde.

**Table 2: Kinetic parameters of KsCcr WT and the KsCcrH365N variant. Values are reported as mean value ± standard error. Calculated with K=1000 for the equilibrium between formaldehyde and its methylene diol form.**

| | | | |
|---|---|---|---|
| 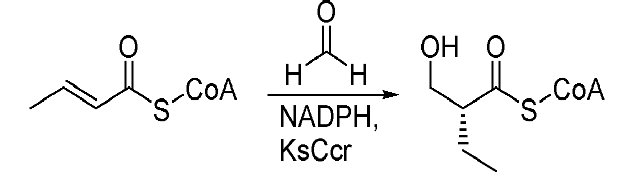 | | | |

| **KsCcr variant** | **Substrate** | **K_{M} (µM)** | **k_{cat} (s⁻¹)** |
|---|---|---|---|
| **Wild-type (WT)** | Crotonyl-CoA | 33 ± 4 | 30 ± 1 |
| | NADPH | 389 ± 49 | |
| | formaldehyde | 32 ± 8 | |
| **His365Asn** | Crotonyl-CoA | 69 ± 17 | 3.1± 0.2 |
| | NADPH | 16 ± 7 | |
| | formaldehyde | 66 ± 23 | |

Analysis of the His365Asn variant had shown that the reaction mechanism of KsCcr with formaldehyde differs from the enzyme's native carboxylation reaction.

To obtain more insights into the catalytic mechanism of reductive aldol reactions, different variants of active site residues were analyzed, which had been described to play an essential role in the carboxylation reaction, using two different formaldehyde concentrations (Table 3).

**Table 3: Ratio of 2-HMB-CoA formed over total products by KsCcr in the presence of two different formaldehyde concentrations. Values were measured in triplicates and are reported as mean value ± standard deviation.**

| **KsCcr variant** | **5 mM** | **75 mM** |
|---|---|---|
| **WT** | 72 ± 1± | 96 ± 0 |
| **Asn81Leu** | 35 ± 0 | 64 ± 2 |
| **Asn81Asp** | 1 ± 0 | 11 ± 0 |
| **Thr82Asp** | 14 ± 1 | 57 ± 1 |
| **Ser119Ala** | 37 ± 3 | 83 ± 0 |
| **Phe170Ala** | 17 ± 0 | 64 ± 0 |
| **Glu171Ala** | 43 ± 0 | 89 ± 0 |
| **His365Asn** | 42 ± 1 | 89 ± 0 |

An essential active site residue for the carboxylation reaction is Asn81, which positions the CO₂ electrophile during catalysis. Substitution of Asn81 by Leu completely impaired the carboxylation of crotonyl-CoA, resulting in the accumulation of a C2-ene adduct that is not further processed by the enzyme and slowly converted into the side product butyryl-CoA. In contrast, variant Asn81 Leu was still able to produce up to 64% of 2-HMB-CoA, indicating that Asn81 is less critical for the reductive aldol reaction. This result was further supported by an experiment, in which variant Asn81 Leu was incubated in the absence of a CO₂ or formaldehyde electrophile, until the C2-ene adduct had been formed. While the addition of CO₂ did not lead to resolution of the adduct, as expected, the addition of formaldehyde resulted in the formation of 2-HMB-CoA. Overall, these results confirmed our calculations that had identified His365 and Glu171, and not Asn81, as being primarily responsible for the enzyme's interaction with formaldehyde.

Residues Thr82 and Ser119 are responsible for the correct orientation of the Asn81 sidechain for a productive interaction with CO₂. When these interactions were compromised, the production of 2-HMB-CoA decreased only to 57% (Thr82Asp) and 83% (Ser119Ala), respectively, at 75 mM formaldehyde, further supporting the notion that in contrast to the reaction with CO₂, the interaction of formaldehyde with Asn81 is not essential for the reductive aldol reaction.

Another important residue in reductive carboxylation is Phe170, which shields the active site from water to suppress the competing protonation reaction. Variant Phe170Ala was strongly compromised in reductive carboxylation (17%), while the same mutant still showed still up to 64% formation of reductive aldol product 2-HMB-CoA at 75 mM formaldehyde. This is likely due to the electrophilic character and the dipolar nature of formaldehyde that allows the molecule to orient in the presence of electric fields and effectively compete with protonation. Notably, an increase of negative charge at the active site reduced production of 2-HMB-CoA, as observed for variant Thr82Asp and to a more significant extend in variant Asn81Asp (Table 3). In summary, the inventors showed that the non-native substrate formaldehyde exploits the active site chemistry of KsCcr very differently than the natural substrate CO₂.

Thus, in further preferred embodiments the polypeptide capable of catalyzing reductive aldol reactions preferably may be a wild type crotonyl-CoA carboxylase/reductase comprising at least 95% sequence identity to amino acid sequence SEQ ID NO: 1. More preferably, the polypeptide capable of catalyzing reductive aldol reactions may comprise the amino acid sequence of SEQ ID NO: 19, SEQ ID NO: 20, SEQ ID NO: 21, SEQ ID NO: 22, SEQ ID NO: 23, SEQ ID NO: 24.

Thus, in preferred embodiments the polypeptide capable of catalyzing reductive aldol reactions may comprise the amino acid sequence of SEQ ID NO: 19:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSNFANYREAFEANRLV
AKGKIHPTLSKVYALEETGQAALDVHHNKHQGKVGVLCLAPREGLGVTDPELRS
KHLTKINAFRNV.

Thus, in preferred embodiments the polypeptide capable of catalyzing reductive aldol reactions may comprise the amino acid sequence of SEQ ID NO: 20:

MQEILDAILSGDAASADYAALALPESYRAVTLHKGEERMFDGLASRDKDPRKSLH
LDDVPLPELGPGEALVAVMASSVNYLTVWSSIFEPVSTFGFLERYGRLSPLTARH
DLPYHVLGSDLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSHFANYREAFEANRLV
AKGKIHPTLSKVYALEETGQAALDVHHNKHQGKVGVLCLAPREGLGVTDPELRS
KHLTKINAFRNV.

Thus, in preferred embodiments the polypeptide capable of catalyzing reductive aldol reactions may comprise the amino acid sequence of SEQ ID NO: 21:

MQEILDAILSGDAASADYAALALPESYRAVTLHKGEERMFDGLASRDKDPRKSLH
LDDVPLPELGPGEALVAVMASSVNYNDVWSSIFEPVSTFGFLERYGRLSPLTAR
HDLPYHVLGSDLAGVVLRTGAGVNAWKPGDEVVAHCLSVELESPDGHNDTMM
DPEQRIWGFETNFGGLAQLALVKTNQLLPKPKHLTWEEAASPGLVNSTAYRQLV
SRNGAGLKQGDNVLIWGASGGLGSYATQYALAGGATPICVVSSPRKADICRAMG
AEAIIDRSAEGYRFWKDEHHQDPREWKRLGGKIREFTGGEDVDIVFEHPGRETF
GASVYVTRKGGTIVTCASTSGYMHQYDNRYLWMSLKRIVGSHFANYREAFEAN
RLVAKGKIHPTLSKVYALEETGQAALDVHHNKHQGKVGVLCLAPREGLGVTDPE
LRSKHLTKINAFRNV

Thus, in preferred embodiments the polypeptide capable of catalyzing reductive aldol reactions may comprise the amino acid sequence of SEQ ID NO: 22:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGADLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSHFANYREAFEANRLV
AKGKIHPTLSKVYALEETGQAALDVHHNKHQGKVGVLCLAPREGLGVTDPELRS
KHLTKINAFRNV.

Thus, in preferred embodiments the polypeptide capable of catalyzing reductive aldol reactions may comprise the amino acid sequence of SEQ ID NO: 23:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH

DLPYHVLGSDLAGVVLRTGAGVNAWKPGDEVVAHCLSVELESPDGHNDTMMDP
EQRIWGAETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSHFANYREAFEANRLV
AKGKIHPTLSKVYALEETGQAALDVHHNKHQGKVGVLCLAPREGLGVTDPELRS
KHLTKINAFRNV.

Thus, in preferred embodiments the polypeptide capable of catalyzing reductive aldol reactions may comprise the amino acid sequence of SEQ ID NO: 24:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRIWGFATNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWMSLKRIVGSHFANYREAFEANRLV
AKGKIHPTLSKVYALEETGQAALDVHHNKHQGKVGVLCLAPREGLGVTDPELRS
KHLTKINAFRNV.

In further preferred embodiments, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 1, wherein the amino acid sequence of SEQ ID NO: 1 comprises modification in the amino acid sequence sections of SEQ ID NO: 3, SEQ ID NO: 5, SEQ ID NO: 7, or SEQ ID NO: 17. More preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 1, wherein the amino acid sequence of SEQ ID NO: 1 comprises modification in the amino acid sequence sections of SEQ ID NO: 5, SEQ ID NO: 7, or SEQ ID NO: 17. Still more preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 1, wherein the amino acid sequence of SEQ ID NO: 1 comprises modification in the amino acid sequence sections of SEQ ID NO: 7, or SEQ ID NO: 17. Preferably the modification is performed by an amino acid substitution.

In further preferred embodiments, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 2, wherein the amino acid sequence of SEQ ID NO: 2 comprises modification in the amino acid sequence sections of SEQ ID NO: 4, SEQ ID NO: 6, SEQ ID NO: 8, or SEQ ID NO: 18. More preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 2, wherein the amino acid sequence of SEQ ID NO: 2 comprises modification in the amino acid sequence sections of SEQ ID NO: 6, SEQ ID NO: 8, or SEQ ID NO: 18. Still more preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 2, wherein the amino acid sequence of SEQ ID NO: 2 comprises modification in the amino acid sequence sections of SEQ ID NO: 8, or SEQ ID NO: 18. Preferably the modification is performed by an amino acid substitution.

The variants of KsCcr WT as mentioned above have the advantage that they do still perform reductive aldol reaction albeit at a much lower rate than KsCcr WT. In addition to this, the reduction side reaction also produces an undesired side product. Thus, although the variants of KsCcr WT as mentioned are slower than the KsCcr WT, they can be more favorable under certain conditions. However, in several embodiments, the enzymatic-catalyzed reductive aldol reaction may be still performed in the absence of CO₂.

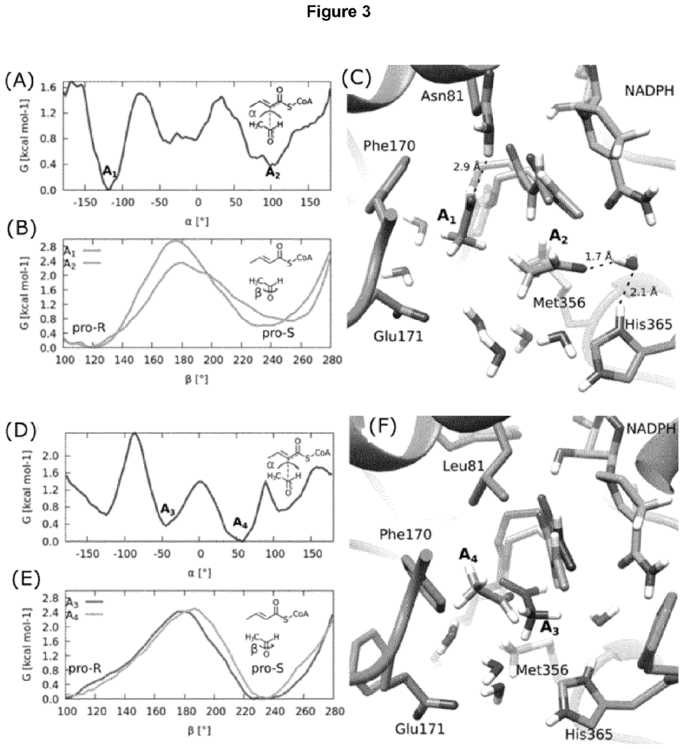

In a further embodiment, the inventors hypothesized that the observed diastereoselectivity with acetaldehyde as carbonyl acceptor originates from preferred binding modes of acetaldehyde with a specific orientation to the crotonyl-CoA substrate. By rotating the pro-R orientation in the active site with umbrella sampling simulations two binding modes were identified represented by free energy minima (Figure 3). In the first binding mode, acetaldehyde is oriented similar to formaldehyde along the dipole moment created by His365 and Glu171 with its methyl group pointing to Met365. In the second binding mode, the carbonyl oxygen of acetaldehyde forms a hydrogen bond with Asn81. Interestingly, in both binding modes, the pro-R conformation is more stable with respect to the pro-S conformation, in line with the observed diastereomeric ratio (Figure 3C). To further increase selectivity for (2S,3R)-EHB-CoA, is was aimed to reduce unfavorable interactions between Met356 and acetaldehyde in the first binding mode by creating variants Met356Ala and Met356Val, which indeed improved the dr for (2S,3R)-EHB-CoA up to 92:8.

To promote formation of the opposite diastereomer (2S,3S)-EHB-CoA, it was aimed at reducing the enzyme's diastereoselectivity by eliminating the interactions of Asn81 with acetaldehyde that favor the pro-R conformation in the second binding mode. Molecular dynamics simulations of variant Asn81 Leu rendered a new orientation of acetaldehyde, in which the molecules oxygen atom would form a hydrogen bond with the NH group of the crotonyl-CoA substrate. This would destabilize hydrogen-bonding of acetaldehyde with Asn81, making the pro-S conformation equally stable compared to the pro-*R* conformation in all binding modes of the Asn81 Leu mutant (Figure 3f). Testing variant Asn81 Leu increased production of (2S,3S)-EHB-CoA to a *dr* of 44:56, essentially eliminating and even slightly inverting the stereochemical outcome of the reaction (see table 4). In conclusion, the inventors engineered the active site of KsCcr to catalyze reductive aldol reactions at high stereoselectivity for 2*S*,3*R* products, and demonstrated that stereoselectivity can also be engineered to favor formation of 2*S*,3*S* products.

**Table 4: Diastereomeric ratio of (2S,3R)-EHB-CoA to (2S,3S)-EHB-CoA formed by KsCcr WT and different variants from crotonyl-CoA and acetaldehyde^{a} . Note that the scheme does not show formation of side product butyryl-CoA. ^{a} Reactions were performed in 100 mM K₂HPO₄ pH = 8 in the presence of 500 mM acetaldehyde for 30 min at 30°C.^{b} Diasteromeric ratio (dr) is given as (2S,3R)-EHB-CoA:(2S,3S)-EHB-CoA.**

| 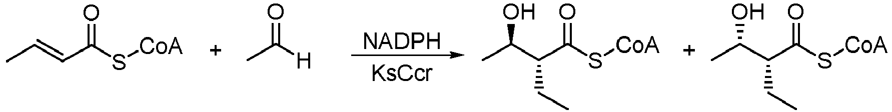 | |
|---|---|
| **KsCcr variant** | **dr***^{b}* |
| **WT** | 82:17 |
| **Asn81Leu** | 44:56 |
| **Ile167Ala** | 94:6 |
| **Met356Ala** | 90:10 |
| **Met365Val** | 92:8 |

Thus, in further preferred embodiments the polypeptide capable of catalyzing reductive aldol reactions preferably may be a wild type crotonyl-CoA carboxylase/reductase comprising at least 95% sequence identity to amino acid sequence SEQ ID NO: 1. More preferably, the polypeptide capable of catalyzing reductive aldol reactions may comprise the amino acid sequence of SEQ ID NO: 25, SEQ ID NO: 26, SEQ ID NO: 27.

Thus, in preferred embodiments the polypeptide capable of catalyzing reductive aldol reactions may comprise the amino acid sequence of SEQ ID NO: 25:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRAWGFETNFGGLAQLALVKTNQLLPKPKHLTWEEAASPGLVNSTAYRQLVS
RNGAGLKQGDNVLIWGASGGLGSYATQYALAGGATPICVVSSPRKADICRAMGA
EAIIDRSAEGYRFWKDEHHQDPREWKRLGGKIREFTGGEDVDIVFEHPGRETFG
ASVYVTRKGGTIVTCASTSGYMHQYDNRYLWMSLKRIVGSHFANYREAFEANRL
VAKGKIHPTLSKVYALEETGQAALDVHHNKHQGKVGVLCLAPREGLGVTDPELR
SKHLTKINAFRNV.

Thus, in preferred embodiments the polypeptide capable of catalyzing reductive aldol reactions may comprise the amino acid sequence of SEQ ID NO: 26:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE
AIIDRSAEGYRFWKDEHHQDPREWKRLGGKIREFTGGEDVDIVFEHPGRETFGA
SVYVTRKGGTIVTCASTSGYMHQYDNRYLWASLKRIVGSHFANYREAFEANRLV
AKGKIHPTLSKVYALEETGQAALDVHHNKHQGKVGVLCLAPREGLGVTDPELRS
KHLTKINAFRNV.

Thus, in preferred embodiments the polypeptide capable of catalyzing reductive aldol reactions may comprise the amino acid sequence of SEQ ID NO: 27:

MQEILDAILSGDAASADYAALALPESYRAVTLHKGEERMFDGLASRDKDPRKSLH
LDDVPLPELGPGEALVAVMASSVNYNTVWSSIFEPVSTFGFLERYGRLSPLTARH
DLPYHVLGSDLAGVVLRTGAGVNAWKPGDEVVAHCLSVELESPDGHNDTMMDP
EQRIWGFETNFGGLAQLALVKTNQLLPKPKHLTWEEAASPGLVNSTAYRQLVSR
NGAGLKQGDNVLIWGASGGLGSYATQYALAGGATPICVVSSPRKADICRAMGAE

AIIDRSAEGYRFWKDEHHQDPREWKRLGGKIREFTGGEDVDIVFEHPGRETFGA
SVYYVTRKGGTIVTCASTSGYMHQYDNRYLWVSLKRIVGSHFANYREAFEANRLV
AKGKIHPTLSKVYALEETGQAALDVHHNKHQGKVGVLCLAPREGLGVTDPELRS
KHLTKINAFRNV.

In further preferred embodiments, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 1, wherein the amino acid sequence of SEQ ID NO: 1 comprises modifications in the amino acid sequence sections of SEQ ID NO: 7, or SEQ ID NO: 30, wherein the amino acid sequence of SEQ ID NO: 30 is WMSLKRIVGSHFAN. More preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 1, wherein the amino acid sequence of SEQ ID NO: 1 comprises a modification in the amino acid sequence section of SEQ ID NO: 7. Preferably the modification is performed by an amino acid substitution.

In further preferred embodiments, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 2, wherein the amino acid sequence of SEQ ID NO: 2 comprises a modification in the amino acid sequence sections SEQ ID NO: 8, or SEQ ID NO: 31, wherein the amino acid sequence of SEQ ID NO: 31 is WMRQKRVQGSHFAN. More preferably, the polypeptide capable of catalyzing reductive aldol reactions may be a modified crotonyl-CoA carboxylase/reductase derived from a wild type crotonyl-CoA carboxylase/reductase comprising the amino acid sequence of SEQ ID NO: 2, wherein the amino acid sequence of SEQ ID NO: 2 comprises a modification in the amino acid sequence section of SEQ ID NO: 8.

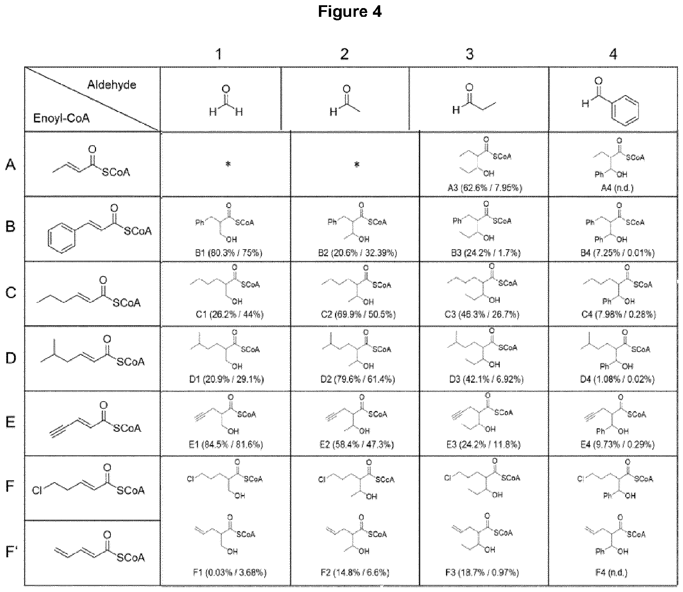

According to phylogenetic and structural analysis, KsCcr belongs to the ECR-1 subfamily of enzymes that are mainly restricted to short-chain enoyl-CoAs. To increase the substrate scope of reductive aldol reactions, the inventors sought to use a more promiscuous enzyme. To that end, a triple variant of *Caulobacter crescentus* crotonyl-CoA carboxylase/reductase (CcCcrC146P/I169A/F373G, here CcCcr_{PAG} ) was employed that had been engineered to accept a variety of enoyl-CoAs, including short- and long-chain, branched, aromatic, bulky and halogenated analogs. Six different enoyl-CoA substrates (Figure 4) were synthesized and tested in combination with four different aldehydes (formaldehyde, acetaldehyde, propionaldehyde, benzaldehyde) and by LC-MS.

Notably, CcCcr_{PAG} catalyzed conversion of all the tested enoyl-CoAs yielding 26 novel aldol products, demonstrating that the protein's active site was able to accommodate the whole range of tested enoyl-CoAs, including the previously not tested 4-in-pentenoyl-CoA (Figure 4, compound **E**). With 5-chloropentenoyl-CoA, we observed a rapid depletion of the CoA-ester in the initial minutes of the time course of the reaction. This was caused by the dechlorination to 2,4-pentadienyl-CoA (Figure 4, compound **F'**) which was also converted by the enzyme.

In respect to promiscuity of CcCcr_{PAG} with aldehydes, it was noted noted that the observed conversion to aldol product decreased with larger substitutions (Figure 4, compounds **3**, **4**). As the enzyme natively accommodates CO₂ at the active site, it is conceivable that substituents larger than C2 are likely sterically too demanding limiting more efficient catalysis without further engineering. In summary, these experiments showed that CcCcr_{PAG} is a highly versatile enzyme that catalyzes the reductive aldol reaction between different enoyl-CoA substrates and aldehyde electrophiles expanding the potential portfolio of products, providing an interesting template for further enzyme engineering campaigns.

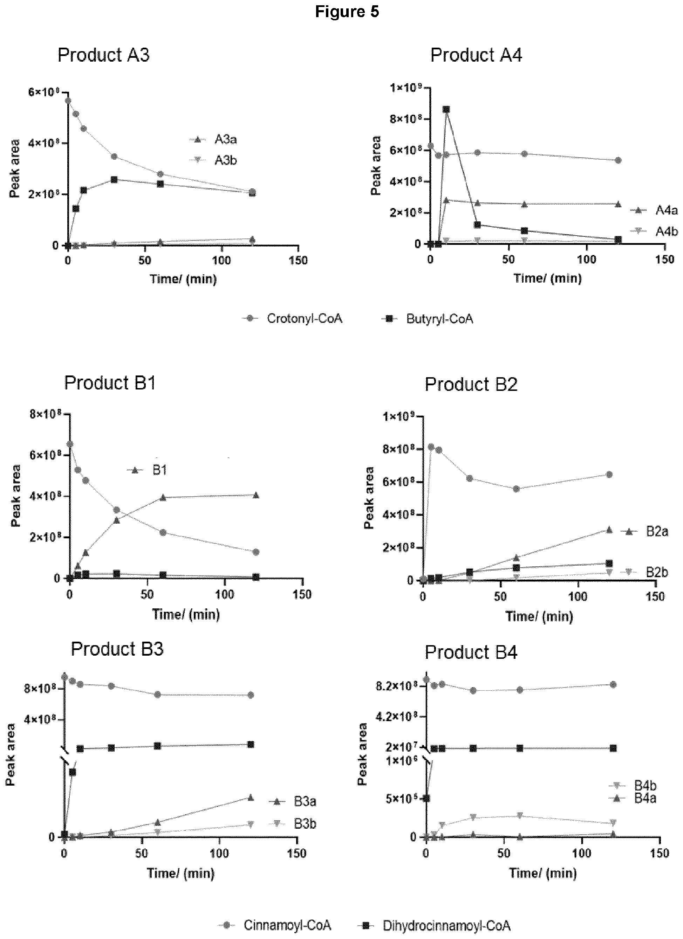

The time course of reactions of the substrate promiscuity screen for compounds A3, A4, B1, B, B3 and B4 of Figure 4, C1, C2, C,3, C4, D1, D2, D3 and D4 of Figure 4, E1, E2, E3, E4, F1, F2, F3 and F4 of Figure 4, and F'1, F'2, F'3 of Figure 4 are shown in Fig. 5, Fig. 6, Fig. 7 and Fig. 8.

Thus, in further preferred embodiments the polypeptide capable of catalyzing reductive aldol reactions preferably may be a wild type crotonyl-CoA carboxylase/reductase comprising at least 95% sequence identity to amino acid sequence SEQ ID NO: 2. More preferably, the polypeptide capable of catalyzing reductive aldol reactions may comprise the amino acid sequence of SEQ ID NO: 29.

Thus, in preferred embodiments the polypeptide capable of catalyzing reductive aldol reactions may comprise the amino acid sequence of SEQ ID NO: 29:

MTIQTLETTALKDLYEIGEIPPAFHVPKTMYAWSIRKERHGKPTQAMQVEVVPTW
EIGEDEVLVLVMAAGVNYNGVWAALGEPISPLDGHKQPFHIAGSDASGIVWKVG

AKVKRWKLGDEVVIHPNQDDGDDEECNGGDPMFSSSQRAWGYETPDGSFAQF
CRVQSRQLLPRPKHLTWEESACYTLTLATAYRMLFGHKPHELKPGQNVLVWGA
SGGLGVFATQLAAVAGANAIGVVSSEDKREFVLSMGAKAVLNRGEFNCWGQLP
KVNGPEFNDYMKESRKFGKAIWQITGNKDVDMVFEHPGEQTFPVSVFLVKRGG
MVVICAGTTGFNLTMDARFLWMRQKRVQGSHGANLMQASAANQLVIDRRVDPC
LSEVFPWDQIPAAHEKMLANQHLPGNMAVLVCAQRPGLRTFEEVQELSGAP.

Thus in further preferred embodiments, the α,β-unsaturated carboxylic acid has the general formula (I): 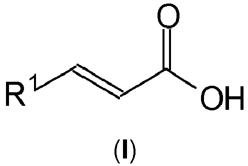 wherein R¹ is selected from hydrogen, an optionally substituted alkyl, alkenyl, alkynyl, alkoxy, carboxy, aminocarbonyl, thiocarbonyl, heteroalkyl, heteroalkenyl, heteroalkynyl, carboxyalkyl, aminoalkyl, haloalkyl, alkylthioalkyl, cycloalkyl, aryl, arylalkyl, heterocycloalkyl, heteroaryl, and heteroarylalkyl.

More preferably, the α,β-unsaturated carboxylic acid has the general formula (I): 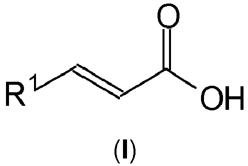 wherein R¹ represents -H, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -C₂H₄Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -C₇H₁₅, -C₈H₁₇, -C(CH₃)₂-C₃H₇, -CH(CH₃)-CH(CH₃)-C₂H₅,-CH₂-CH(CH₃)-CH(CH₃)₂, -CH₂-C(CH₃)₂-C₂H₅, -CH(CH₃)-C(CH₃)₃, -C(CH₃)₂-CH(CH₃)₂, -C₂H₄-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₂H₄-CH=CH₂, -CH=CH-C₂H₅, -CH=C(CH₃)₂, -CH₂-CH=CH-CH₃, -CH=CH-CH=CH₂, C₃H₆-CH=CH₂, -CH=CH-C₃H₇, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -CH₂-C=CH, -C=CH, -C≡C-CH₃, -C₂H₄-C≡CH, -C≡C-C₂H₅, -CH₂-C≡C-CH₃, -C≡C-CH=CH₂, -CH=CH-C=CH, -C=C-C=CH, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₄H₃-C≡CH, -C≡C-C₄H₉, -C₃H₆-C≡C-CH₃, -CH₂-C≡C-C₃H₇, -C₂H₄Ph, -CH=CH-Ph, -C=C-Ph, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -CH₂R², -CH₂CH₂R², or -CH₂CH₂CH₂R²; and R² represents -NH₂, -OH, -SH, -F, -CI, -Br, -I, -CN, -N₃, -OCN, -NCO, -SCN, or -NCS.

More preferably, R¹ represents -H, -Ph, -CH₂-Ph, -C₂H₄Ph, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -C(CH₃)₂-C₃H₇, -CH(CH₃)-CH(CH₃)-C₂H₅, -CH₂-CH(CH₃)-CH(CH₃)₂, -CH₂-C(CH₃)₂-C₂H₅, -CH(CH₃)-C(CH₃)₃, -C(CH₃)₂-CH(CH₃)₂, -C₂H₄-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C₂H₄-CH=CH-CH₃, -CH₂-CH=CH-C₂H₅, -CH₂-CH=C(CH₃)₂, -C(CH₃)=C(CH₃)₂, -C₂H₄-CH=CH₂, -CH=CH-C₂H₅, -CH=C(CH₃)₂, -CH₂-CH=CH-CH₃, -C₃H₆-CH=CH₂, -CH=CH-C₃H₇, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -CH₂-C=CH, -C=CH, -C≡C-CH₃, -C₂H₄-C≡CH, -C≡C-C₂H₅, -CH₂-C≡C-CH₃, -C₃H₆-C≡CH, -C≡C-C₃H₇, -C₂H₄-C≡C-CH₃, -CH₂-C≡C-C₂H₅, -C₄H₈-C≡CH, -C≡C-C₄H₉, -C₃H₆-C=C-CH₃, -CH₂-C≡C-C₃H₇, -CH=CH-Ph, -C=C-Ph, -C₆H₄-OCH₃, -CH₂-CH₂-OCH₃, -CH₂-OCH₃, -CH₂-C₆H₄-OCH₃, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS.

Still more preferably, R¹ represents -H, -Ph, -CH₂-Ph, -C₂H₄Ph, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -CH₂-C=CH, -C=CH, -C≡C-CH₃, -C₂H₄-C≡CH, -C≡C-C₂H₅, -CH₂-C≡C-CH₃, -C₃H₆-C≡CH, -C≡C-C₃H₇, -C₂H₄-C≡C-CH₃, -CH₂-C≡C-C₂H₅, -C₄H₈-C≡CH, -C≡C-C₄H₉, -C₃H₆-C≡C-CH₃, -CH₂-C≡C-C₃H₇, -CH=CH-Ph, -C=C-Ph, -CH₂-CH₂-OCH₃, -CH₂-OCH₃, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS.

Even more preferably, R¹ represents -H, -Ph, -CH₂-Ph, -C₂H₄Ph, -CH₃, -C₂H₅, -C₃H₇, -CH(CH₃)₂, -CH=CH₂, -CH₂-CH=CH₂, -C(CH₃)=CH₂, -CH=CH-CH₃, -CH₂-C=CH, -C=CH, -C≡C-CH₃, -C₂H₄-C≡CH, -C≡C-C₂H₅, -CH₂-C≡C-CH₃, -C₃H₆-C≡CH, -C≡C-C₃H₇, -C₂H₄-C≡C-CH₃, -CH₂-C≡C-C₂H₅, -C₄H₈-C≡CH, -C≡C-C₄H₉, -C₃H₆-C≡C-CH₃, -CH₂-C≡C-C₃H₇, -CH=CH-Ph, -C=C-Ph, -CH₂-CH₂-OCH₃, -CH₂-OCH₃, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS.

Still more preferably, R¹ represents -H, -Ph, -CH₂-Ph, -C₂H₄Ph, -CH₃, -C₂H₅, -C₃H₇, -CH=CH₂, -CH₂-CH=CH₂, -CH=CH-CH₃, -CH₂-C=CH, -C=CH, -C≡C-CH₃, -CH=CH-Ph, -C=C-Ph, -CH₂R², -CH₂CH₂R², or -CH₂CH₂CH₂R²; and
R² represents -F, -CI, -Br, -I.

In particular preferred embodiments, the α,β-unsaturated carboxylic acid may be selected from the group comprising or consisting of acrylic acid, crotonic acid (*trans*-2-butenoic acid), *trans*-cinnamic acid, 5-chloro-2-pentenoic acid, *trans-2-*hexenoic acid, 5-methyl-2-hexenoic acid, *trans*-2-penten-4-ynoic acid and penta-2,4-dienoic acid. More preferred is acrylic acid or crotonic acid. Most preferred is crotonic acid.

In further preferred embodiments, the carbonyl acceptor has the general formula (II): 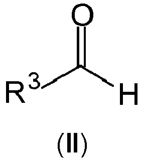 wherein R³ represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -Ph, or -CH₂-Ph.

Preferably, R³ represents -H, -CH₃, -C₂H₅, -C₃H₇, -CH(CH₃)₂, -C₄H₉, -CH₂-CH(CH₃)₂, -CH(CH₃)-C₂H₅. More preferably, R³ represents -H, -CH₃, -C₂H₅, -C₃H₇, -C₄H₉. More preferably, R³ represents -H, -CH₃, -C₂H₅, -C₃H₇. More preferably, R³ represents -H, -CH₃, -C₂H₅. Thus, it is preferred that the carbonyl acceptor is selected from the group comprising or consisting of formaldehyde, acetaldehyde and propionaldehyde. Most preferably, R³ represents -H, or -CH₃. Thus, most preferred is that the carbonyl acceptor is formaldehyde or acetaldehyde.

In further preferred embodiments, the method may further comprise a cleavage of the coenzyme A thioester to obtain the free form of the α-branched β'-hydroxy carbonyl compound. Thus, in preferred embodiments the method may further comprise performing a hydrolysis reaction with the coenzyme A thioester of the α-branched β'-hydroxy carbonyl compound under basic conditions or by using a thioesterase.

Thus, the method of the present invention preferably further comprises the following step:
d) performing a hydrolysis reaction with the coenzyme A thioester of the α-branched β'-hydroxy carbonyl compound under basic conditions or by using a thioesterase (E.C.3.1.2.).

Therefore, the present invention preferably further relates to a method, wherein the free form of the α-branched β'-hydroxy carbonyl compound has the general formula (**IIIa**) and the α-branched β'-hydroxyacyl-CoA has the general formula (**IIIb**): 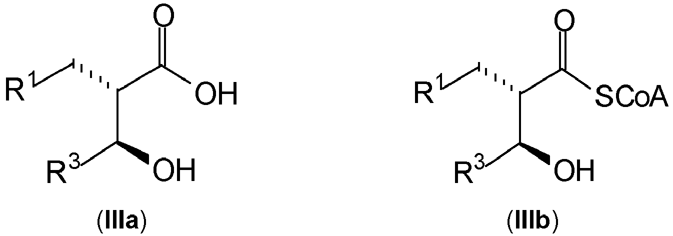 and R¹, R², and R³ have the same meanings as defined herein.

Thus, the method of the present invention preferably further comprises the following step:
e) isolating the α-branched β'-hydroxy carbonyl compound or the α-branched β'-hydroxyacyl-CoA thiosester, wherein the α-branched β'-hydroxy carbonyl compound has the general formula (**IIIa**) and the α-branched β'-hydroxyacyl-CoA has the general formula (**IIIb**): 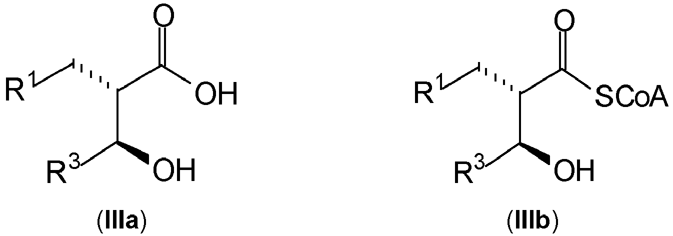 and R¹, R², and R³ have the same meanings as defined herein.

In a further preferred embodiment, the enzyme-catalyzed reductive aldol reaction may be performed in the presence of an acyl-CoA oxidase. The acyl-CoA oxidase can be used to continually recycle the side product alkyl-CoA back into the α,β-unsaturated carbonyl donor.

In further embodiments, the enzymatic-catalyzed reductive aldol reaction is performed in the absence of CO₂. However, CO₂ does not have to be completely avoided and running the reactions at ambient conditions is possible.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
the α,β-unsaturated carboxylic acid has the general formula (I): 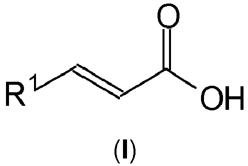 wherein R¹ represents -H, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -C₂H₄Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -C₇H₁₅, -C₈H₁₇, -C(CH₃)₂-C₃H₇, -CH(CH₃)-CH(CH₃)-C₂H₅, -CH₂-CH(CH₃)-CH(CH₃)₂, -CH₂-C(CH₃)₂-C₂H₅, -CH(CH₃)-C(CH₃)₃, -C(CH₃)₂-CH(CH₃)₂, -C₂H₄-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₂H₄-CH=CH₂, -CH=CH-C₂H₅, -CH=C(CH₃)₂, -CH₂-CH=CH-CH₃, -CH=CH-CH=CH₂, C₃H₆-CH=CH₂, -CH=CH-C₃H₇, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -CH₂-C≡CH, -C=CH, -C≡C-CH₃, -C₂H₄-C≡CH, -C≡C-C₂H₅, -CH₂-C≡C-CH₃, -C≡C-CH=CH₂, -CH=CH-C=CH, -C=C-C=CH, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C≡CH, -C≡C-C(CH₃)=CH₂, -C₄H₃-C≡CH, -C≡C-C₄Hg, -C₃H₆-C≡C-CH₃, -CH₂-C≡C-C₃H₇, -C₂H₄Ph, -CH=CH-Ph, -C=C-Ph, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS; and
wherein the carbonyl acceptor has the general formula (**II**): 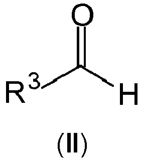 wherein R³ represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -Ph, or -CH₂-Ph.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
the α,β-unsaturated carboxylic acid has the general formula (**I**): 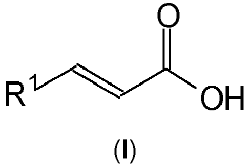 wherein R¹ represents -H, -Ph, -CH₂-Ph, -C₂H₄Ph, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -CH₂-C≡CH, -C=CH, -C≡C-CH₃, -C₂H₄-C≡CH, -C≡C-C₂H₅, -CH₂-C≡C-CH₃, -C₃H₆-C≡CH, -C≡C-C₃H₇, -C₂H₄-C≡C-CH₃, -CH₂-C≡C-C₂H₅, -C₄H₈-C≡CH, -C≡C-C₄H₉, -C₃H₆-C≡C-CH₃, -CH₂-C≡C-C₃H₇, -CH=CH-Ph, -C=C-Ph, -CH₂-CH₂-OCH₃, -CH₂-OCH₃, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS, and
wherein the carbonyl acceptor has the general formula (**II**): 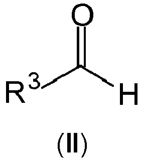 wherein R³ represents -H, -CH₃, -C₂H₅, -C₃H₇, -C₄H₉.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
the α,β-unsaturated carboxylic acid has the general formula (**I**): 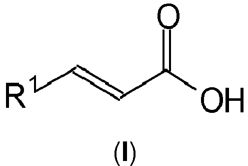 wherein R¹ represents -H, -Ph, -CH₂-Ph, -C₂H₄Ph, -CH₃, -C₂H₅, -C₃H₇, -CH(CH₃)₂, -C₄H₉, -CH₂-CH(CH₃)₂, -CH(CH₃)-C₂H₅, -C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -CH₂-C≡CH, -C=CH, -C≡C-CH₃, -C₂H₄-C≡CH, -C≡C-C₂H₅, -CH₂-C≡C-CH₃, -C₃H₆-C≡CH, -C≡C-C₃H₇, -C₂H₄-C≡C-CH₃, -CH₂-C≡C-C₂H₅, -C₄H₈-C≡CH, -C≡C-C₄H₉, -C₃H₆-C≡C-CH₃, -CH₂-C≡C-C₃H₇, -CH=CH-Ph, -C=C-Ph, -CH₂-CH₂-OCH₃, -CH₂-OCH₃, -CH₂R², -CH₂CH₂R², or -CH₂CH₂CH₂R²; and
R² represents -NH₂, -OH, -SH, -F, -CI, -Br, -I, -CN, -N₃, -OCN, -NCO, -SCN, or -NCS, and
wherein the carbonyl acceptor has the general formula (**II**): 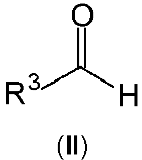 wherein R³ represents -H, -CH₃, -C₂H₅, -C₃H₇, or -C₄H₉,
wherein the α-branched β'-hydroxy carbonyl compound has the general formula (**IIIa**) and the α-branched β'-hydroxyacyl-CoA has the general formula (**IIIb**): 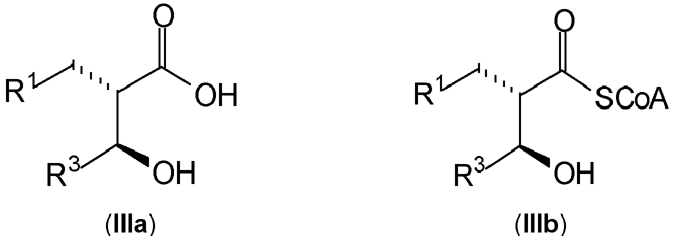 and R¹, R², and R³ have the same meanings as defined above.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide comprises at least 95% sequence identity to amino acid sequence SEQ ID NO: 1 or SEQ ID NO:2, preferably the polypeptide comprises at least 95% sequence identity to amino acid sequence SEQ ID NO: 1.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the polypeptide comprises at least 95% sequence identity to amino acid sequence SEQ ID NO: 1 or SEQ ID NO:2, preferably the polypeptide comprises at least 95% sequence identity to amino acid sequence SEQ ID NO: 1.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide comprises the amino acid sequence SEQ ID NO: 19, SEQ ID NO: 20, SEQ ID NO: 21, SEQ ID NO: 22, SEQ ID NO: 23 or SEQ ID NO: 24.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the polypeptide comprises the amino acid sequence SEQ ID NO: 19, SEQ ID NO: 20, SEQ ID NO: 21, SEQ ID NO: 22, SEQ ID NO: 23 or SEQ ID NO: 24.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide comprises the amino acid sequence SEQ ID NO: 25, SEQ ID NO: 26 or SEQ ID NO: 27.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the polypeptide comprises the amino acid sequence SEQ ID NO: 25, SEQ ID NO: 26 or SEQ ID NO: 27.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide comprises the amino acid sequence SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the polypeptide comprises the amino acid sequence SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the polypeptide is a wild type crotonyl-CoA carboxylase/reductase.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase is obtained from *Kitasatospora setae* or *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase is obtained from *Kitasatospora setae.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase is obtained from *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the cofactor is NADPH.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase; and
wherein the cofactor is NADPH.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a wild type crotonyl-CoA carboxylase/reductase; and wherein the cofactor is NADPH.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the cofactor is NADPH,
wherein the polypeptide is a wild type crotonyl-CoA carboxylase/reductase; and wherein the polypeptide is a crotonyl-CoA carboxylase/reductase is obtained from *Kitasatospora setae* or *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the cofactor is NADPH,
wherein the polypeptide is a wild type crotonyl-CoA carboxylase/reductase; and wherein the polypeptide is a crotonyl-CoA carboxylase/reductase is obtained from *Kitasatospora setae.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase;
wherein the polypeptide comprises at least 95% sequence identity to amino acid sequence SEQ ID NO: 1 or SEQ ID NO:2, preferably the polypeptide comprises at least 95% sequence identity to amino acid sequence SEQ ID NO: 1; and
wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a wild type crotonyl-CoA carboxylase/reductase, wherein the α,β-unsaturated carboxylic acid is selected from acrylic acid and crotonic acid, preferably crotonic acid.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the carbonyl acceptor is selected from the group comprising or consisting of formaldehyde, acetaldehyde and propionaldehyde, preferably formaldehyde and acetaldehyde.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a wild type crotonyl-CoA carboxylase/reductase, wherein the polypeptide is a crotonyl-CoA carboxylase/reductase obtained from from *Kitasatospora setae* or *Caulobacter crescentus,* preferably *Kitasatospora* setae,
wherein the α,β-unsaturated carboxylic acid is selected from acrylic acid and crotonic acid, preferably crotonic acid,
wherein the carbonyl acceptor is selected from the group comprising or consisting of formaldehyde, acetaldehyde and propionaldehyde, preferably formaldehyde and acetaldehyde, wherein the cofactor is NADPH, and

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase;
wherein the α,β-unsaturated carboxylic acid is selected from the group comprising or consisting of crotonic acid (*trans*-2-butenoic acid), *trans*-cinnamic acid, 5-chloro-2-pentenoic acid, *trans*-2-hexenoic acid, 5-methyl-2-hexenoic acid, *trans*-2-penten-4-ynoic acid and penta-2,4-dienoic acid, and
wherein the carbonyl acceptor is selected from the group comprising or consisting of formaldehyde, acetaldehyde and propionaldehyde.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
wherein the polypeptide is a crotonyl-CoA carboxylase/reductase;
wherein the α,β-unsaturated carboxylic acid is selected from the group comprising or consisting of crotonic acid (*trans*-2-butenoic acid), *trans*-cinnamic acid, 5-chloro-2-pentenoic acid, *trans*-2-hexenoic acid, 5-methyl-2-hexenoic acid, *trans*-2-penten-4-ynoic acid and penta-2,4-dienoic acid, and
wherein the carbonyl acceptor is selected from the group comprising or consisting of formaldehyde, acetaldehyde and propionaldehyde,
wherein the polypeptide is a crotonyl-CoA carboxylase/reductase obtained from *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide comprises at least 95% sequence identity to amino acid sequence SEQ ID NO: 1 or SEQ ID NO:2, preferably the polypeptide comprises at least 95% sequence identity to amino acid sequence SEQ ID NO: 1.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide comprises at least 95% sequence identity to amino acid sequence SEQ ID NO: 1 or SEQ ID NO:2, preferably the polypeptide comprises at least 95% sequence identity to amino acid sequence SEQ ID NO: 1.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide comprises the amino acid sequence SEQ ID NO: 19, SEQ ID NO: 20, SEQ ID NO: 21, SEQ ID NO: 22, SEQ ID NO: 23 or SEQ ID NO: 24.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide comprises the amino acid sequence SEQ ID NO: 19, SEQ ID NO: 20, SEQ ID NO: 21, SEQ ID NO: 22, SEQ ID NO: 23 or SEQ ID NO: 24.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide comprises the amino acid sequence SEQ ID NO: 25, SEQ ID NO: 26 or SEQ ID NO: 27.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide comprises the amino acid sequence SEQ ID NO: 25, SEQ ID NO: 26 or SEQ ID NO: 27.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide comprises the amino acid sequence SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide comprises the amino acid sequence SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase; and
wherein the polypeptide is a wild type crotonyl-CoA carboxylase/reductase.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase is obtained from *Kitasatospora setae* or *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase is obtained from *Kitasatospora setae.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase is obtained from *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the cofactor is NADPH.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the cofactor is NADPH,
wherein the polypeptide is a wild type crotonyl-CoA carboxylase/reductase; and wherein the polypeptide is a crotonyl-CoA carboxylase/reductase is obtained from *Kitasatospora setae* or *Caulobacter crescentus.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the cofactor is NADPH,
wherein the polypeptide is a wild type crotonyl-CoA carboxylase/reductase; and wherein the polypeptide is a crotonyl-CoA carboxylase/reductase is obtained from *Kitasatospora setae.*

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase;
wherein the polypeptide comprises at least 95% sequence identity to amino acid sequence SEQ ID NO: 1 or SEQ ID NO: 2, preferably the polypeptide comprises at least 95% sequence identity to amino acid sequence SEQ ID NO: 1; and
wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a wild type crotonyl-CoA carboxylase/reductase, wherein the polypeptide is a crotonyl-CoA carboxylase/reductase obtained from from *Kitasatospora setae* or *Caulobacter crescentus,* preferably *Kitasatospora* setae,
wherein the α,β-unsaturated carboxylic acid is selected from acrylic acid and crotonic acid, preferably crotonic acid,
wherein the carbonyl acceptor is selected from the group comprising or consisting of formaldehyde, acetaldehyde and propionaldehyde, preferably formaldehyde and acetaldehyde,
wherein the cofactor is NADPH, and

Therefore, the present invention preferably relates to a method for preparing an α-branched β'-hydroxy carbonyl compound by reductive aldol reaction, wherein the method comprises the following steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase,
d) performing a hydrolysis reaction with the coenzyme A thioester of the α-branched β'-hydroxy carbonyl compound under basic conditions or by using a thioesterase
wherein the polypeptide is a crotonyl-CoA carboxylase/reductase;
wherein the α,β-unsaturated carboxylic acid is selected from the group comprising or consisting of crotonic acid (*trans*-2-butenoic acid), *trans*-cinnamic acid, 5-chloro-2-pentenoic acid, *trans*-2-hexenoic acid, 5-methyl-2-hexenoic acid, *trans*-2-penten-4-ynoic acid and penta-2,4-dienoic acid, and
wherein the carbonyl acceptor is selected from the group comprising or consisting of formaldehyde, acetaldehyde and propionaldehyde.

### Kits

The enoyl-CoA carboxylases/reductases or reductive aldolase polypeptides used in the method according to the present invention for preparing α-branched β'-hydroxy carbonyl compound by reductive aldol reaction may be provided in the form of kits. The enzymes in the kits may be present individually or as a plurality of enzymes. The kits can further include reagents for carrying out the enzymatic reactions, substrates for assessing the activity of enzymes, as well as reagents for detecting the products. The kits can also include reagent dispensers and instructions for use of the kits.

The kits described herein can include arrays comprising a plurality of different polypeptides having the enzymatic activity of a polypeptide capable of catalyzing reductive aldol reactions at different addressable positions, wherein the different polypeptides are different variants of a reference sequence each having at least one different improved enzyme property.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide is an enoyl-CoA carboxylase/reductase.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide is a crotonyl-CoA carboxylase/reductase.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises at least 95% sequence identity to amino acid sequence SEQ ID NO: 1 or SEQ ID NO:2.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

In one embodiment the kit for preparing an α-branched β'-hydroxy carbonyl compound by reductive aldol reaction further comprises the cofactor NADPH.

Thus, the present invention is also directed to a kit for preparing an α-branched β'-hydroxy carbonyl compound by reductive aldol reaction comprising:
a) a polypeptide capable of catalyzing reductive aldol reactions in the presence of a cofactor, wherein the polypeptide is an enoyl-CoA carboxylase/reductase,
b) NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide is a crotonyl-CoA carboxylase/reductase,
b) NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises at least 95% sequence identity to amino acid sequence SEQ ID NO: 1 or SEQ ID NO:2,
b) NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29,
b) NADPH.

Thus, the present invention is also directed to a kit for preparing an α-branched β'-hydroxy carbonyl compound by reductive aldol reaction comprising:
a) a polypeptide capable of catalyzing reductive aldol reactions in the presence of a cofactor, wherein the polypeptide is an enoyl-CoA carboxylase/reductase,
b) NADPH,
c) a cofactor generation system for the conversion of NADP⁺ to NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide is a crotonyl-CoA carboxylase/reductase,
b) NADPH,
c) a cofactor generation system for the conversion of NADP⁺ to NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises at least 95% sequence identity to amino acid sequence SEQ ID NO: 1 or SEQ ID NO:2,
b) NADPH,
c) a cofactor generation system for the conversion of NADP⁺ to NADPH.

In one embodiment the kit for preparing an α-branched β'-hydroxy carbonyl compound by reductive aldol reaction,
comprises:
a) a polypeptide capable of catalyzing reductive aldol reactions in the presence of a cofactor, wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29,
b) NADPH,
c) a cofactor generation system for the conversion of NADP⁺ to NADPH.

A description of the sequences of SEQ ID NO:1 to SEQ ID NO:36 as disclosed herein can be found in the following table 5.

**Table 5: Description of Sequences as disclosed herein.**

| | |
|---|---|
| SEQ ID NO: 1 | Kitasatospora setae KsCCR crotonyl-CoA carboxylase/reductase |
| SEQ ID NO: 2 | *Caulobacter crescentus* CcCCR crotonyl-CoA carboxylase/reductase |
| SEQ ID NO: 3 | KsCcr crotonyl-CoA carboxylase/reductase amino acid residues 68-84 |
| SEQ ID NO: 4 | CcCcr crotonyl-CoA carboxylase/reductase amino acid residues 61-77 |
| SEQ ID NO: 5 | KsCcr crotonyl-CoA carboxylase/reductase amino acid residues 113-145 |
| SEQ ID NO: 6 | CcCcr crotonyl-CoA carboxylase/reductase amino acid residues 93-125 |
| SEQ ID NO: 7 | KsCcr crotonyl-CoA carboxylase/reductase amino acid residues 165-172 |
| SEQ ID NO: 8 | CcCcr crotonyl-CoA carboxylase/reductase amino acid residues 146-153 |
| SEQ ID NO: 9 | KsCcr crotonyl-CoA carboxylase/reductase amino acid residues 187-201 |
| SEQ ID NO: 10 | CcCcr crotonyl-CoA carboxylase/reductase amino acid residues 168-182 |
| SEQ ID NO: 11 | KsCcr crotonyl-CoA carboxylase/reductase amino acid residues 208-214 |
| SEQ ID NO: 12 | CcCcr crotonyl-CoA carboxylase/reductase amino acid residues 190-195 |
| SEQ ID NO: 13 | KsCcr crotonyl-CoA carboxylase/reductase amino acid residues 227-258 |
| SEQ ID NO: 14 | CcCcr crotonyl-CoA carboxylase/reductase amino acid residues 208-239 |
| SEQ ID NO: 15 | KsCcr crotonyl-CoA carboxylase/reductase amino acid residues 310-319 |
| SEQ ID NO: 16 | CcCcr crotonyl-CoA carboxylase/reductase amino acid residues 296-305 |
| SEQ ID NO: 17 | KsCcr crotonyl-CoA carboxylase/reductase amino acid residues 363-368 |
| SEQ ID NO: 18 | CcCcr crotonyl-CoA carboxylase/reductase amino acid residues 347-354 |
| SEQ ID NO: 19 | KsCcr variant His365Asn |
| SEQ ID NO: 20 | KsCcr variant Asn81 Leu |
| SEQ ID NO: 21 | KsCcr variant Thr82Asp |
| SEQ ID NO: 22 | KsCcr variant Ser119Ala |
| SEQ ID NO: 23 | KsCcr variant Phe170Ala |
| SEQ ID NO: 24 | KsCcr variant Glu171Ala |
| SEQ ID NO: 25 | KsCcr variant IIe167AIa |
| SEQ ID NO: 26 | KsCcr variant Met356Ala |
| SEQ ID NO: 27 | KsCcr variant Met356Val |
| SEQ ID NO: 28 | I176A Forward Primer 5'->3' |
| SEQ ID NO: 29 | CcCcr C125P/I148A/F352G |
| SEQ ID NO: 30 | KsCCR crotonyl-CoA carboxylase/reductase amino acid residues 355-368 |
| SEQ ID NO: 31 | CcCCR crotonyl-CoA carboxylase/reductase amino acid residues 339-354 |
| SEQ ID NO: 32 | Reverse Primer 5'->3' |
| SEQ ID NO: 33 | M356A Forward Primer 5'->3' |
| SEQ ID NO: 34 | Reverse Primer 5'->3' |
| SEQ ID NO: 35 | Forward Primer 5'->3' |
| SEQ ID NO: 36 | Reverse Primer 5'->3' |

### Description of the Figures

**Figure 1** shows reactions catalyzed by enoyl-CoA carboxylase/reductase (Ecr). Initial reduction of **1** with NADPH yields an enolate intermediate that can further react with different electrophiles to yield **2**, **3** and **4.** Reaction with CO₂ yields **4**, the enzyme's native reaction product. Reducing or omitting the concentration of CO₂ in the reaction leads to formation of (side) product **2.** The present invention demonstrates that replacing the native substrate CO₂ by different aldehydes advantageously gives rise to **3.**
**Figure 2** shows a reaction mechanism for the reductive aldol reaction between crotonyl-CoA (brown) and formaldehyde (FALD) in KsCcr obtained from QM/MM molecular dynamics simulations (A) Free energy profile for the reductive aldol reaction with formaldehyde. Representative snapshots of the reactant state (B), the transition state (C), the protonation of the alkoxy reactive species by the conserved water molecule (D), the proton transfer from His365 (E), and the 2-HMB-CoA product (F). The reaction mechanism's various steps are identified in the free energy profile and displayed in panels B-F with characteristic average distances between crotonyl-CoA, NADPH, FALD, a conserved water molecule, and His365.
**Figure 3** shows preferred binding conformations of acetaldehyde in the active site of KsCcr wild type (left) and KsCcrN81L (right). (A) Free energy profile for different orientations of the pro-*R* conformation in the active site of wt KsCcr obtained from umbrella sampling simulation of the angle α. The preferred binding conformations are two minima A₁ and A₂ characterized by a hydrogen bond with the side chain of Asn81 or alignment along the electric field created by the negatively charged side chain of Glu171 and protonated His365 (representative snapshot of the active site shown in (C). (B) Free energy change of transforming the pro-*R* to the pro-*S* conformation for the two preferred binding modes A₁ and A₂ with the angle α. In both cases pro-*R* is the favored conformation in agreement with measured diastereoselectivity. (C) Representative snapshot of the wt active site. (D) Free energy profile of the pro-*R* conformation in the active site of the Asn81Leu variant. The most stable conformations A₃ and A₄ differ from wild type KsCcr displaying a hydrogen bond to the NH group of the crotonyl substrate and no specific interactions to the enzyme, respectively (see representative snapshot in (F). (E) Free energy change of transforming the pro-*R* in the pro-*S* conformation for the two minima A₃ and A₄ in the Asn81Leu variant with the angle α. The free energy profile render both conformations equally favorable in line with the measured reduced diastereoselectivity. (F) Representative snapshot of the Asn81Leu variant's active site.
**Figure 4** shows the reductive aldol reaction between different enoyl-CoAs and aldehydes catalyzed by CcCcr_{PAG}- The matrix shows the products formed by reductive aldol reaction catalyzed by CcCcrPAG between enoyl-CoAs (A-F') and aldehydes (1-4). Values in parenthesis report the % conversion of substrates A-F (X_{A-F}) and the % selectivity of aldol product A1-F4 (S_{A1-F4}) after 2h incubation. Values for row F summarize products detected for F and F' as substrates. *Combinations not included in the screen. n.d. not detected.
**Figure 5** shows the time course of reactions of the substrate promiscuity screen for compounds A3, A4, B1, B, B3 and B4 of Figure 4.. Peak area corresponds to the area in the EIC.

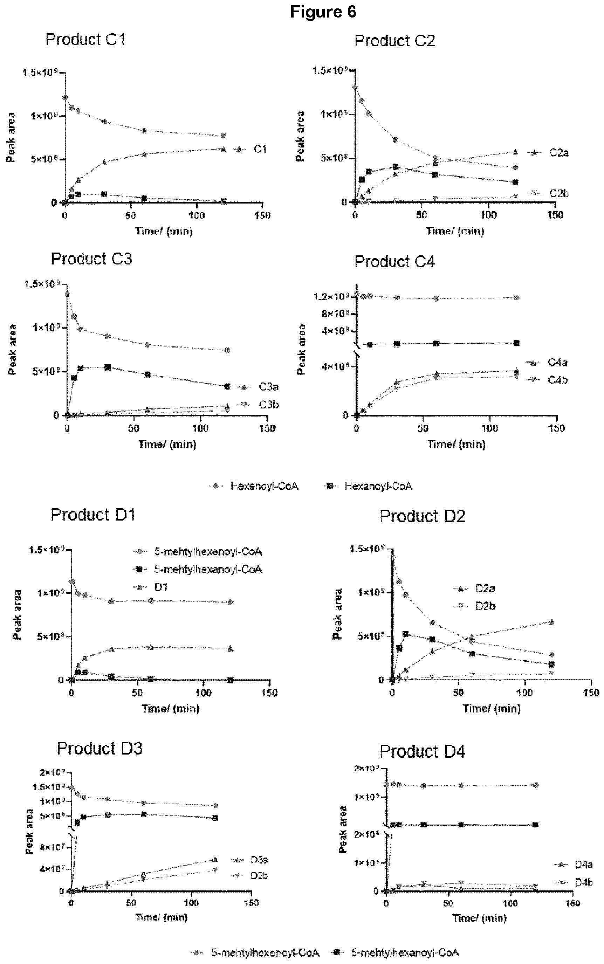

**Figure 6** shows the time course of reactions of the substrate promiscuity screen for compounds C1, C2, C,3, C4, D1, D2, D3 and D4 of Figure 4. Peak area corresponds to the area in the EIC.

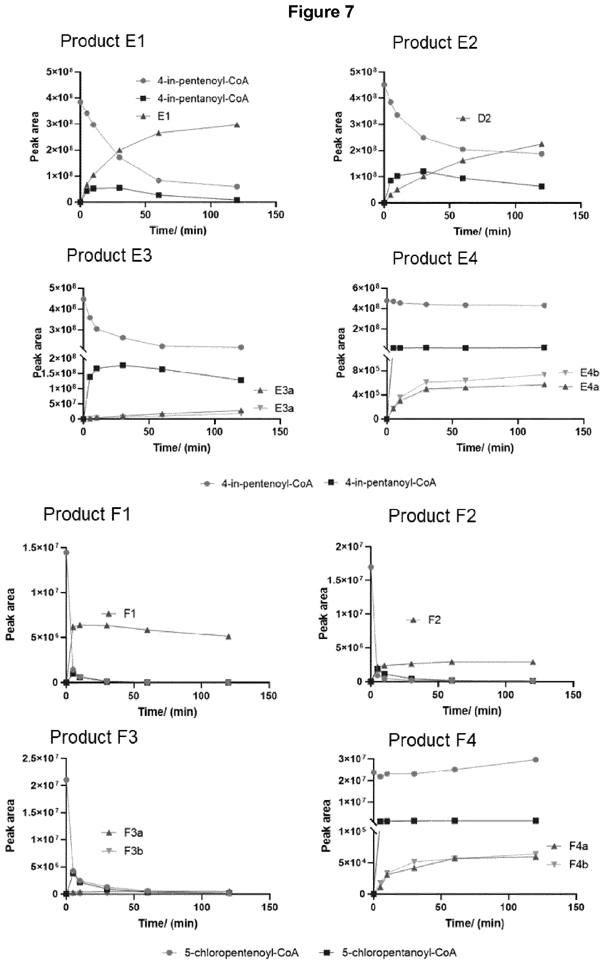

**Figure 7** shows the time course of reactions of the substrate promiscuity screen for compounds E1, E2, E3, E4, F1, F2, F3 and F4 of Figure 4. Peak area corresponds to the area in the EIC.

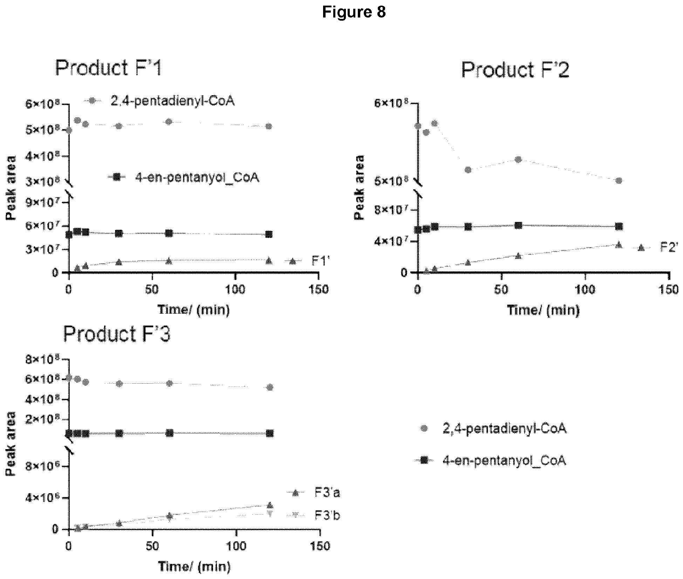

**Figure 8** shows the time course of reactions of the substrate promiscuity screen for compounds F'1, F'2, F'3 of Figure 4. Peak area corresponds to the area in the EIC.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Materials and Methods

### Chemicals

Crotonic Anhydride, formaldehyde solution 37%, acetaldehyde, hexanoic acid, 5-methylhexanoic acid, 4-pentynoic acid, trans-cinnamic acid and 5-chloropentanoic acid were purchased from Sigma Aldrich AG. Coenzyme A trilithium salt and DNAse I were purchased from Roche Diagnostics and NADPH Na₄ (98%) from Carl Roth GmbH. Solvents and salts were all analytical grade or better. Crotonyl-CoA was synthesized according to Peter, D.M., Vögeli, B., Cortina, N.S. & Erb, T.J. A Chemo-Enzymatic Road Map to the Synthesis of CoA Esters. Molecules 21, 517 (2016).

### Synthesis of Reference Compounds (2S,3S)-2-Ethyl-3-hydroxybutanoic acid and (2S,3R)-2-Ethyl-3-hydroxybutanoic acid.

All non-aqueous reactions were carried out using flame-dried glassware under argon atmosphere. All solvents were distilled by rotary evaporation. Solvents for non-aqueous reactions were dried as follows prior to use: Tetrahydrofuran (THF) was dried with potassium hydroxide and subsequently distilled from Solvona^{®}. Methylene chloride, di*i*sopropylamine and di*i*sopropylethylamine were distilled from calcium hydride. All other commercially obtained reagents were used as received.

Reactions were monitored by thin layer chromatography (TLC) using Merck Silica Gel 60 F₂₅₄-plates and visualised by fluorescence quenching under UV-light. In addition, TLC-plates were stained using a potassium permanganate or cerium sulfate/phosphomolybdic acid stain. Chromatographic purification of products was performed on Merck Silica Gel 60 (230-400 mesh) unless otherwise noted using a forced flow of eluents. Concentration *in vacuo* was performed by rotary evaporation at 40 °C and appropriate pressure and by exposing to fine vacuum at room temperature if necessary.

NMR spectra were recorded on a Bruker AV 300 MHz, AV III 500 MHz, AV III HD 500 MHz spectrometer at room temperature. Chemical shifts are reported in ppm with the solvent resonance as internal standard. Data are reported as follows: s = singlet, bs = broad singlet, d = doublet, t = triplet, q = quartet, quint = quintet, sext = sextet, m = multiplet. Diastereomers are featured with a/b, rotamers with */**.

Mass spectra were recorded by the mass service department of the Philipps-Universität Marburg. HR-EI, ESI or APCI mass spectra were acquired with an LTQ-FT mass spectrometer (Thermo Fischer Scientific). The resolution was set to 100 000.

IR spectra were recorded on a Bruker IFS 200 spectrometer. The absorption bands are given in wave numbers (cm⁻¹), intensities are reported as follows: s = strong, m = medium, w = weak, br = broad band.

Optical rotations were determined at 20 °C for Na-D wavelength (589 nm) with a Krüss P8000-T polarimeter.

### (S)-4-Benzyl-3-butyryloxazolidin-2-one

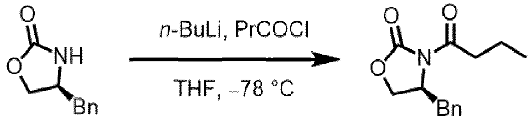

To a solution of (*S*)-oxazolidinone (2.00 g, 11.3 mmol, 1.00 eq.) in tetrahydrofuran (25 mL) at -78 °C, under an atmosphere of argon was added a solution of *n*-butyllithium in hexane (2.5 _{M}, 4.74 mL, 11.9 mmol, 1.05 eq.) dropwise over 10 min. The mixture was stirred for 15 min and butyryl chloride (1.28 mL, 12.4 mL, 1.10 eq.) was added dropwise over 10 min. After 3 h at -78 °C a solution of sat. ammonium chloride solution was added. The mixture was extracted with ethyl acetate (3x) and the combined extracts were dried (magnesium sulfate). After removal of the solvent *in vacuo* the residue was purified by flash chromatography (n-pentane/ethyl acetate 5:1) to yield the oxazolidinone product (2.50 g, 10.1 mmol, 90%) as colourless oil. **R*_{f}*** = 0.80 (n-pentane/ethyl acetate 1:1); **¹H-NMR:** (300 MHz, CDCl₃): *δ* = 7.37-7.20 (m, 5H, Ph-CH), 4.43 (ddd, 1H, ³*J* = 13.4, 7.0, 3.5 Hz, NC*H*), 4.24-4.14 (m, 2H, Ph-C*H*₂), 3.31 (dd, 1H, ²*J* = 13.4 Hz, ³*J* = 3.5 Hz, O-C*H*₂), 3.04-2.72 (m, 3H, O-C*H*₂, C*H*₂CH₂CH₃), 1.74 (sext, 2H, CH₂C*H*₂CH₃), 1.02 (t, 3H, ³*J* = 7.4 Hz, CH₃) ppm. The analytical data is in accordance to literature. ^{[1]}

### (S)-4-Benzyl-3-((2S,3R)-2-ethyl-3-hydroxybutanoyl)oxazolidin-2-one

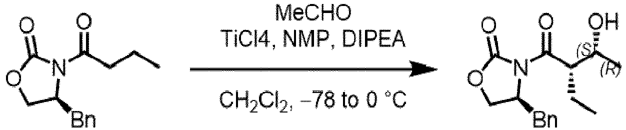

To a solution of the oxazolidinone (0.50 g, 2.02 mmol, 1.00 eq.) in methylene chloride (10 mL) at 0 °C, under an atmosphere of argon was added titanium tetrachloride (0.24 mL, 2.22 mmol, 1.10 eq.) dropwise. After 15 min di*iso*propylethylamine (0.53 mL, 3.03 mmol, 1.50 eq.) was added over a period of 5 min, and the mixture was stirred for 45 min. *N*-methylpyrrolidone (0.21 mL, 2.22 mmol, 1.10 eq.) was added and after 10 min acetaldehyde (0.23 mL, 4.04 mmol, 2.00 eq.). After 2 h half sat. ammonium chloride solution was added and the mixture extracted with methylene chloride (3x). The combined extracts were dried (magnesium sulfate) and the solvent was removed *in vacuo.* The residue was purified by flash chromatography (n-pentane/ethyl acetate 5:1 to 1:1) to give the hydroxy oxazolidinone product (0.49 g, 1.72 mmol, 85%) as yellow oil. **R***_{f}* = 0.50 (n-pentane/ethyl acetate 1:1); **¹H-NMR:** (500 MHz, CDCl₃): *δ* = 7.37-7.22 (m, 5H, Ph-CH), 4.74 (ddd, 1H, ³*J* = 13.3, 7.0, 3.5 Hz, NCH), 4.22-4.13 (m, 2H, Ph-C*H*₂), 4.13-4.06 (m, 1H, CHOH), 3.54 (dt, 1H, ³*J* = 9.4, 4.5 Hz, C*H*CH₂), 3.37 (dd, 1H, ²*J* = 13.3, ³*J* = 3.2 Hz, OC*H*₂), 2.71 (dd, 1H, ²*J* = 13.3, ³*J* = 10.0Hz, OC*H*₂), 2.46 (bs, O*H*), 1.96-1.81 (m, 1H, C*H*₂CH₃), 1.77-1.64 (m, 1H, C*H*₂CH₃), 1.23 (d, 3H, ³*J* = 6.4 Hz, CHC*H*₃), 0.91 (t, 3H, ³*J* = 7.5 Hz, CH₂C*H*₃) ppm; **¹³C-NMR:** (125 MHz, CDCl₃): *δ* = 175.5 (N*C*=O), 153.9 (*C*O₂), 135.2 (Ph-*C*_{q}), 129.3 (2×Ph-*C*H₂), 129.0 (2×Ph-*C*H₂), 127.4 (Ph-*C*H), 68.6 (*C*HOH), 66.3 (Ph-*C*H₂), 55.6 (N*C*H), 50.1 (*C*HCH₂), 38.1 (O*C*H₂), 20.7 (*C*H₂CH₃), 19.5 (CH*C*H₃), 11.9 (CH₂*C*H₃) ppm; **FT-IR:** *U*ₘₐₓ(film): 3482 (w), 3029 (w), 2969 (w), 2932 (w), 2877 (w), 1774 (s), 1691 (m), 1604 (w), 1455 (w), 1384 (m), 1350 (w), 1288 (w), 1272 (w), 1208 (s), 1151 (w), 1114 (m), 1077 (w), 1050 (w), 1017 (w), 983 (w), 948 (w), 910 (w), 822 (w), 763 (w), 746 (w), 702 (m), 593 (w), 572 (w), 506 (w) cm⁻¹.

### (2S,3R)-2-Ethyl-3-hydroxybutanoic acid

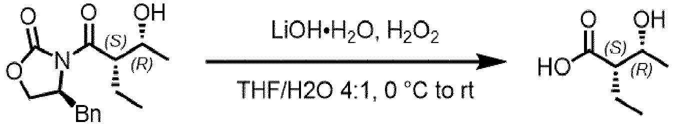

To a solution of the oxazolidinone (0.23 g, 0.79 mmol, 1.00 eq.) in a 4:1 mixture of tetrahydrofuran/water (5 mL) at 0 °C was added a solution of hydrogen peroxide (30% in water, 0.21 mL, 2.05 mmol, 2.60 eq.) and lithium hydroxide monohydrate (66 mg, 1.58 mmol, 2.00 eq.). The mixture was stirred overnight reaching room temperature. After careful addition of a sat. solution of sodium thiosulfate at 0 °C, the mixture was extracted with ethyl acetate (3x). The mixture was extracted with dichloromethane (3x) and the pH value of the aqueous layer was adjusted (pH = 2-3) with hydrochloric acid (6 _{M}) and extracted with ethyl acetate (4x). The combined extracts were dried (sodium sulfate), filtered and the volatiles removed *in vacuo.* The residue was purified by flash chromatography (ethyl acetate) to yield the product (50 mg, 0.38 mmol, 48%) as clear oil. **R***_{f}* = 0.15 (ethyl acetate); **¹H-NMR:** (500 MHz, CDCl₃): *δ* = 4.07 (t, 1H, ³*J* = 5.4 Hz, CHOH), 2.44-2.39 (m, 1H, C*H*CH₂), 1.77-1.71 (m, 1H, C*H*₂CH₃), 1.70-1.61 (m, 1H, C*H*₂CH₃), 1.25 (d, 3H, ³*J* = 6.3 Hz, CHC*H*₃), 1.00 (t, 3H, ³*J* = 7.4 Hz, CH₂C*H*₃) ppm. (O*H* and CO₂*H* were not detectable due to rapid exchange with the solvent); **¹³C-NMR:** (125 MHz, CDCl₃): 179.7 (*C*O₂H), 68.0 (CHOH), 53.5 (*C*HCH₂), 20.3 (*C*H₂CH₃), 20.1 (CH*C*H₃), 12.2 (CH₂*C*H₃) ppm; **FT-IR:** *U*ₘₐₓ(film): 3348 (w), 2969 (m), 2933 (w), 2880 (w), 1704 (s), 1461 (w), 1409 (w), 1382 (w), 1263 (w), 1201 (m), 1152 (w), 1086 (m), 1051 (w), 1021 (w), 954 (w), 910 (w), 886 (w), 782 (w), 732 (m), 662 (w), 521 (w), 485 (w) cm⁻¹; **HRMS (ESI):** *m*/*z* calcd. for C₆H₁₂O₃Na [M+Na]⁺ 155.0684, found 155.0679; **[*α*]²⁰_{D}:** -5.6° (c = 0.5, chloroform).

### Methyl (2S,3S)-2-ethyl-3-hydroxybutanoate

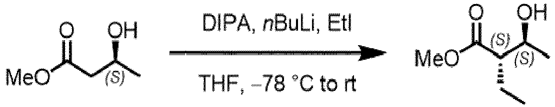

To a solution of the di*iso*propylamine (6.19 mL, 44 mmol, 2.60 eq.) in tetrahydrofuran (30 mL) at 0 °C under an atmosphere of argon was added a solution of *n*-butyllithium in hexane (2.5 _{M}, 16.9 mL, 42.3 mmol, 2.50 eq.) dropwise. After 30 min the solution was cooled to -78 °C and a solution of hydroxy ester (1.87 mL, 16.9 mmol, 1.00 eq.) in tetrahydrofuran (8 mL) was added over a period of 20 min. The solution was stirred for 4 h reaching -30 °C and after recooling to -78 °C a solution of ethyl iodide (5.04 mL, 62.6 mmol, 3.70 eq.) in tetrahydrofuran (7 mL) was added over a period of 20 min. The solution was stirred overnight reaching room temperature. A sat. solution of ammonium chloride was added and the mixture extracted with diethyl ether (3x). The combined extracts were dried (sodium sulfate), filtered and the volatiles carefully removed *in vacuo.* The residue was purified by flash chromatography (n-pentane/diethyl ether 3:1 to 1:1) to yield the ester product (2.40 g, 16.4 mmol, 97%) as pale-yellow liquid. **R***_{f}* = 0.60 (*n*-pentane/ethyl acetate 1:1); **¹H-NMR:** (300 MHz, CDCl₃): *δ* = 3.92 (quint, 1H, ³*J* = 6.3 Hz, C*H*OH), 3.72 (s, 3H, CO₂C*H*₃), 2.33 (dt, 1H, ³*J* = 8.0, 6.3 Hz, C*H*CH₂), 1.75-1.61 (m, 2H, C*H*₂CH₃), 1.22 (d, 3H, ³*J* = 6.4 Hz, CHC*H*₃), 0.92 (t, 3H, ³*J* = 7.5 Hz, CH₂C*H*₃) ppm. (O*H* was not detectable due to rapid exchange with the solvent). The analytical data is in accordance with literature.^{[2]}

### (2S,3S)-2-Ethyl-3-hydroxybutanoic acid

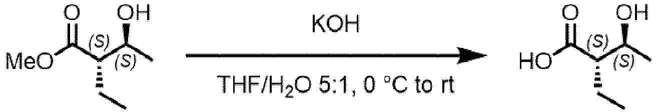

To a solution of the ester (400 mg, 2.74 mmol, 1.00 eq.) in a 5:1 mixture of water/tetrahydrofuran (9 mL) at room temperature was added potassium hydroxide (1.53 g, 27.4 mmol, 10.0 eq.) in one portion. After stirring overnight water was added and the pH-value was adjusted (pH = 3) with hydrochloric acid (1 _{M}). The mixture was extracted with ethyl acetate (3x), the combined extracts dried (magnesium sulfate), filtered and the volatiles were removed *in vacuo.* The residue was purified by flash chromatography (*n*-pentane/ethyl acetate 1:1) to yield the acid product (300 mg, 2.27 mmol, 83%) as pale-yellow oil. **R***_{f}* = 0.15 (ethyl acetate); **¹H-NMR:** (500 MHz, CDCl₃): *δ* = 3.98 (t, 1H, ³*J* = 6.3 Hz, C*H*OH), 2.33 (dt, 1H, ³*J* = 8.0, 6.3 Hz, C*H*CH₂), 1.77-1.65 (m, 2H, C*H*₂CH₃), 1.28 (d, 3H, ³*J* = 6.3 Hz, CHC*H*₃), 0.99 (t, 3H, ³*J* = 7.5 Hz, CH₂C*H*₃) ppm. (O*H* was not detectable due to rapid exchange with the solvent); **¹³C-NMR:** (125 MHz, CDCl₃): 179.8 (*C*O₂H), 68.0 (CHOH), 54.2 (*C*HCH₂), 22.4 (*C*H₂CH₃), 21.4 (CH*C*H₃), 11.6 (CH₂*C*H₃) ppm; **FT-IR:** *U*ₘₐₓ(film): 3366 (w), 2969 (m), 2937 (w), 2880 (w), 1704 (s), 1461 (w), 1383 (w), 1273 (w), 1201 (m), 1147 (w), 1116 (m), 1090 (w), 1058 (w), 1018 (w), 953 (w), 884 (w), 841 (w), 782 (w), 657 (w), 514 (w) cm⁻¹; **HRMS (ESI):** *m*/*z* calcd. for C₆H₁₂O₃Na [M+Na]⁺ 155.0684, found 155.0676; **[*α*]²⁰_{D}:** -4.0° (c = 0.5, chloroform).

### Cloning and mutagenesis

Acyl-CoA ligases RevS and At4CL4 were identified by literature research. Codon optimized genes containing either N- or C-terminal 6x His-tags for RevS and At4CL4, respectively were synthesized and cloned into pET-16b and pET-21a expression vectors by Baseclear.

The KsCcr gene was provided by the JGI. Enzyme variants KsCcrI167A, M356A and M365V variants were generated with the QuikChange^{®} Site-Directed Mutagenesis Kit (Stratagene, La Jolla, USA). Employed primer pairs are reported in Table 6.

**Table 6: Primer pairs employed for the generation of KsCcr variants.**

| **Mutati on** | **Forward Primer 5'->3'** | SEQ ID NO: | **Reverse Primer 5'->3'** | SEQ ID NO: |
|---|---|---|---|---|
| **1176A** | GACCCAGAGCAGCG CGCCTGGGGCTTC GAAAC | SEQ ID NO: 28 | GTTTCGAAGCC CCAGGCGCGCT GCTCTGGGTC | SEQ ID NO: 32 |
| **M356A** | CAACCGTTACCTGTG GGCGTCCCTGAAAC GTATC | SEQ ID NO: 33 | GATACGTTTCA GGGACGCCCAC AGGTAACGGTT | SEQ ID NO: 34 |
| **M356V** | CCGTTACCTGTGGGT GTCCCTGAAAC | SEQ ID NO: 35 | GTTTCAGGGAC ACCCACAGGTA | SEQ ID NO: 36 |

### Gene expression and protein purification

His-tagged KsCcr and its variants were expressed in *E*. *coli* BL21 (DE3). Cells in terrific broth were grown at 37 °C to an OD₆₀₀ = 0.8-1.0 upon which expression for 12-16 h at 23 °C was induced by the addition of 500 µM IPTG (Isopropyl-D-β-thiogalactopyranoside). Cells were harvested for 15 min at 7500x g at 4 °C then resuspended in 2 mL of buffer A (50 mM Tris, 500 mM NaCl, 1M L-Proline, pH= 7.5) per gram of pellet. The suspension was treated with 10 mg/mL of DNAse I, 5 mM MgCl₂, 10 ug/ml of lysozyme and incubated on ice for 20 min upon which cells were lysed using a sonicator. The lysate was clarified at 45000x g at 4°C for 40 min and then loaded onto a pre-equilibrated 1 mL HisTrap FF column and washed with 15 % buffer B (50 mM Tris, 500 mM NaCl, 1 M L-Proline, 500 mM imidazole, pH = 7.5) for 20-30 column volumes until the UV 280 nm reached the baseline level. The protein was eluted by applying 100% buffer B, collected then pooled and desalted into 12.5 mM Tris, 125 mM NaCl, 1 M L-Proline pH = 7.5. The addition of L-Proline to each buffer increased the yields for the protein purification by making the protein more soluble as previously reported. The protein was flash frozen in N₂ (I) and stored at -80°C if not immediately used for assays.

The same procedure was employed for the expression and purification of CcCcr_{PAG} with a modified buffer composition. Buffer A contained 50 mM Tris HCl pH= 7.9, 500 mM NaCl, buffer B contained 50 mM Tris HCl pH= 7.9, 500 mM NaCl and the desalting buffer contained 25 mM Tris HCl pH= 7.9, 125 mM NaCl, 30 % glycerol.

Acyl-CoA oxidase Acx4 was expressed in *E*. *coli* BL21-Al cells (Invitrogen). Cells were grown at 37°C to an OD₆₀₀ = 1 and the expression was induced by the addition of 500 µM IPTG and L-arabinose (0.02 % w/v). Cells were harvested after an additional 6 hours of incubation at 30 °C. The purification procedure followed the same protocol as for KsCcr with buffer A containing 50 mM HEPES pH = 7.6, 500 mM NaCl, buffer B containing 50 mM HEPES pH = 7.6, 500 mM NaCl, 500 mM imidazole and desalting buffer containing 25 mM TrisHCl pH = 7.9, 150 mM NaCl, 30 % glycerol. Additionally, the concentration of FAD in Acx4 batches was calculated using a standard curve for absorption at 450 nm (ε_{FAD 450 nm} = 11.3 mM⁻¹ cm⁻¹) and reconstituted to equimolar concentrations of protein and cofactor.

### Chemical synthesis of (2S,3S)-2-EHB-CoA and (2S,3R)-2-EHB-CoA

Enoyl-CoAs where synthesized using the mixed anhydride method adapted from a known protocol. The unsaturated acid (63 µmol) and triethylamine (70 µmol) were dissolved in CH₂Cl₂ (2 ml) and stirred at 23°C for 30 min. The reaction was cooled to 4 °C and ethylchloroformate (70 µmol) was added. After 2 h the solvent was evaporated and a solution of CoA-trilithium salt (31 µmol) in 0.4 M KHCO₃ (2 ml) was added. The reaction procedure was monitored by mixing 5 µl of reaction mixture with 35 µl of an aqueous 5,5'-dithiobis-2-nitrobenzoic acid (DTNB, Ellman's reagent) solution. Upon completion the reaction was acidified to pH = 3-4 with formic acid, diluted to 35 ml with H₂O and lyophilized. The product was resuspended in H₂O and purified by reverse phase HPLC over a Gemini 10 µm NX-C18 110 Å, 100 × 21.2 mm, AXIA packed column (Phenomenex) using a gradient from 5% to 65% methanol with 25 mM ammonium formate pH=8.1 as the aqueous phase. Fractions containing the product were pooled and lyophilized. Lyophilized fractions were analyzed by HPLC over an Agilent Eclipse Plus C18, 3.5 µm 100 Å, 100 × 2.1 mm, 4.6 × 100 mm column using 5% to 30% acetonitrile gradient over 7.5 min with 25 mM ammonium formate pH=8.1 as the aqueous phase. The fractions containing the expected product were pooled, lyophilized and stored at -20°C if not used.

### Enzymatic synthesis of enoyl-CoAs for the substrate promiscuity screen

Synthesis was carried out using an adopted, one-pot enzymatic synthesis setup. The final assay volume was 6 ml and contained 100 mM HEPES pH = 7.5, 20 mM MgCl₂, 100 mM KHCO₃, 4.35 mM (20 mg) CoA, 17.38 mM ATP and 20.86 mM of the corresponding carboxylic acid. Despite their low solubility in water, cinnamic acid and 5-methylhexanoic acid could be added to the assay directly while still enabling full CoA depletion.

Upon equilibration of the assay mixture at 30 °C, the enzymes were added to a final concentration of 5 µM ligase (At4CL4 for the mixture containing cinnamic acid, RevS for all other setups) and 3 µM Acx4. The reactions were incubated for 2 h 30°C at 200 rpm, while monitoring CoA consumption using Ellman's reagent. Upon completion, the reaction was quenched with a final concentration of 10% (v/v) formic acid, centrifuged for 10 min at 5000 g, filtered through a 0.2 µm syringe filter and flash frozen in N₂ (I) and stored at -80°C if not immediately subjected to HPLC-MS purification.

All enoyl-CoAs were purified via reverse phase LC/MS using a Gemini 10 µm NX-C18 110 Å, 100 × 21.2 mm, AXIA packed column (Phenomenex). Using 50 mM NH₄HCO₂ pH 8.2 as aqueous phase, the column was equilibrated after injection for 2 min with 5 % MeOH, followed by a gradient from 5 % to 40 % MeOH in 19 min, a 2 min washing step at 95 % MeOH and a re-equilibration step of 3 min at 5 % MeOH. The flow rate was kept constant at 25 ml min⁻¹. Fractions containing the product were pooled, flash frozen in liquid N2, lyophilized and stored at -20°C until use. The concentration was determined via UV/Vis at 260 nm assuming an exctinction coefficient of 22.4 mM⁻¹ cm⁻¹. For cinnamoyl-CoA ε_{cinnamoyl-CoA 308nm} =16.6 mM⁻¹ cm⁻¹ was employed.

### Isolation and structural characterization of aldol products by NMR

Synthesis, isolation and preparation of formaldehyde aldol product 2-HMB-CoA:15 mg of crotonyl-CoA, 16 mg of NADPH, were dissolved in 100 mM KH₂PO₄ pH = 8, 100 mM formaldehyde and the reaction started by addition of 10 µM KsCcr in a final volume of 3 mL and incubated at 30 °C for 20 min. The reaction was quenched by addition of 100 µL 50% formic acid and centrifuged at 17000x g to precipitate the protein. 2-HMB-CoA was purified by preparative RPLC/MS over a Gemini 10 µm NX-C18 110 Å, 100 × 21.2 mm, AXIA packed column (Phenomenex) using a methanol gradient from 5 % to 30 % over 10.5 min with 25 mM ammonium formate pH = 8.1 as the aqueous phase. Fractions containing the product were pooled, lyophilized and stored at -20°C. Prior to NMR experiments, 4.7 mg of 2-HMB-CoA were dissolved in 600 µL 50 mM KH₂PO₄ pH = 7.0 and lyophilized. The sample was resuspended in 600 µL of D₂O resulting in a final concentration of 9 mM in 50 mM KD₂PO₄ pD = 7.4 and measured at 25°C, following procedures described earlier.

Synthesis, isolation and preparation of acetaldehyde aldol product 2-EHB-CoA: 10 mg of crotonyl-CoA 80 mg of NADPH, were dissolved in 100 mM KH₂PO₄ pH = 8 with 500 mM acetaldehyde and the reaction started by addition of 2 µM KsCcr in a final volume of 2 mL and incubated at 30 °C for 30 min at which point Acx4, to a final concentration of 24 µM, was added. At 110 min and 3 h reaction time another half equivalent of Acx4 was added to reach a final concentration of 48 µM. The reaction was monitored by HPLC and upon full conversion to 2-EHB-CoA. The reaction was quenched by addition of 100 µL 50% formic acid and lyophilized. The reaction mixture was still contaminated with crotonyl-CoA and was incubated with 4mM NADPH in 100 mM KH₂PO₄ pH = 8 with enoyl reductase Etr1p to reduce the remaining crotonyl-CoA to butyryl-CoA. Upon completion the reaction was quenched with 50% formic acid and centrifuged for 10 min at 17000x g at 4°C to precipitate the protein. 2-EHB-CoA was purified by preparative RPHPLC/MS over a Gemini 10 µm NX-C18 110 Å, 100 × 21.2 mm, AXIA packed column (Phenomenex) using a methanol gradient from 5 % to 30 % over 10.5 min with 25 mM ammonium formate pH = 8.1 as the aqueous phase. Fractions containing the product were pooled, lyophilized and stored at -20°C. Prior to NMR experiments, 0.38 mg of 2-EMB-CoA were dissolved in 600 µL 50 mM KH₂PO₄ pH = 7.0 and lyophilized. The sample was resuspended in 600 µL of D₂O and measured at 25°C, following procedures described earlier.

### Quantification of formaldehyde and acetaldehyde

Formaldeyhde stock solution concentration was determined by following the formation of phenylhydrazone at 368nm ( ε_{Phenylhydrazone,368nm} = 6.1 mM⁻¹ cm⁻¹). Assays were performed on a Cary-60 UV/Vis spectrophotometer (Agilent) at 30°C using quartz cuvettes (10 mm path length; Hellma) contained K₂HPO₄ pH = 8.0 and 3 mM phenylhydrazine.

Acetaldehyde stock solution concentration was determined by its reduction to ethanol using alcohol dehydrogenase adhE from *E.coli* and measuring the depletion of NADPH at 340 nm (ε_{NADPH,340nm} = 6.22 mM⁻¹ cm⁻¹). Assays were performed on a Cary-60 UV/Vis spectrophotometer (Agilent) at 30°C using quartz cuvettes (10 mm path length; Hellma) and contained 100 mM Hepes pH = 7.5, 300 µM NADH and adhE.

### Quantification of 2-HMB-CoA and 2-EHB-CoA

2-HMB-CoA was quantified by incubation with thioesterase YciA from E.Coli and following the reaction of CoA with Ellmann's reagent (5,5'-dithiobis-(2-nitrobenzoic acid) (DTNB)). The reaction releases 2-nitro-5-thiobenzoate (TNB⁻) that ionizes to TNB²⁻. The latter compound was detected spectrophotometrically on a Cary-60 UV/Vis spectrophotometer (Agilent) at 30°C using quartz cuvettes (10 mm path length; Hellma) at a wavelength of 412 nm with ε_{TNB2-,412nm} = 14.15 mM⁻¹ cm⁻¹. For quantification of 2-EHB-CoA, the compound was incubated in 1M NaOH for 15 min at 80°C to hydrolyze the CoA-ester and the solution neutralized using HCl. The released CoA was quantified using Ellmann's reagent as described for 2-HMB-CoA.

### Kinetic characterization

For reactions in which the full conversion to the aldol product was achieved assays were performed on a Cary-60 UV/Vis spectrophotometer (Agilent) at 30°C using quartz cuvettes (1mm, 3mm or 10 mm path length; Hellma). Reactions were performed in 100 mM K₂HPO₄ pH = 8.0. Kinetic parameters for one substrate were determined by varying its concentration while the others were kept constant at 10 times their K_{M} value. Reaction procedure was monitored by following the oxidation of NADPH at 365 nm (ε_{NADPH,365nm} = 3.33 mM⁻¹ cm⁻¹). For reactions where both butyryl-CoA and the aldol product where formed the steady state parameters were determined using a discontinuous assay. Reactions of 100 µL volume were performed in 100 mM K₂HPO₄ pH = 8.0 at 30°C and each concentration was assayed in triplicates. Samples of 20 µL reaction were quenched with 2 µL of 50% formic acid and centrifuged for 10 min at 17'000 g at 4°C. 10 µL of this solution were diluted in 40 µL of 500 mM Tris HCl pH = 7 and incubated overnight at room temperature. Samples were analyzed using by HPLC over an Agilent Eclipse Plus C18, 3.5 µm 100 Å, 100 × 2.1 mm, 4.6 × 100 mm column using 5% to 30% acetonitrile gradient over 7.5 min with 25 mM ammonium formate pH=8.1 as the aqueous phase.

### HPLC analysis of reaction products

Reactions for product analysis contained saturating amounts (at least 10 times the K_{M}) of crotonyl-CoA, NADPH, 100 mM K₂HPO₄ pH = 8.0, and varying amounts of formaldehyde in a final volume of 100 µL. The reaction procedure was monitored by decrease in absorbance of NADPH at 365 nm, quenched with 10 µL of 50% formic acid at completion and spinned at 17'000 g for 10 min to precipitate the protein. The reaction was diluted 10 times into 5% methanol/Buffer 8.1 and analyzed by HPLC over an Agilent Eclipse Plus C18, 3.5 µm 100 Å, 100 × 2.1 mm, 4.6 × 100 mm column using 5% to 30% acetonitrile gradient over 7.5 min with 25 mM ammonium formate pH=8.1 as the aqueous phase.

### Substrate promiscuity screen

100 µL Assays contained 1 mM enoyl-CoA, 5 mM NADPH, 500 mM aldehyde (formaldehyde was employed at 75 mM), 5 µM CcCcr_{PAG}, 3 µM Acx4 and 0.1 µg/µL catalase. Assays were performed in 100 mM K₂HPO₄ pH = 8.0 at 30 °C and 10 µL samples were taken at different time points and quenched with 1 µL of 50 % formic acid. The samples were spinned at 17000 g for 10 min at 4°C to precipitate the protein and then analyzed by LC-MS.

### LC-MS analysis of CoA esters

Determination of CoA esters was performed using a HRES-LC-MS. The chromatographic separation was performed on an Thermo Scientific Vanquish HPLC System using a Kinetex Evo C18 column (50 × .12mm, 100 A, 1.7 µm, Phenomenexc) equipped with a 20 × 2.1 mm guard column of similar specificity at a constant eluent flow rate of 0.3 mL/min and a column temperature of 40 °C with eluent A being 50 mM ammonium formate pH = 8.1 and eluent B being MeOH (Honeywell) The injection volume was 1 µl. The elution profile consisted of the following steps and linear gradients: 0 - 1 min constant at 2.5 % B; 1 - 6 min from 2.5 to 90 % B; 6 - 8 min constant at 90 % B; 8 - 8.1 min from 90 to 2.5 % B; 8.1 - 10 min constant at 2.5 % B. A Thermo Scientific ID-X Orbitrap mass spectrometer was used in positive mode with an electrospray ionization source and the following conditions: ESI spray voltage 3800 V, sheath gas at 60 arbitrary units, auxiliary gas at 15 arbitrary units, sweep gas at 2 arbitrary units, ion transfer tube temperature at 300°C and vaporizer temperature at 300 °C. Scheduled targeted collision induced dissociation was performed on the two suspect molecules according to the table, applying a precursor ion scan at a mass range between 800 and 900 m/z with a mass resolution of 120000 using the orbitrap mass analyzer after quadrupole pre-isolation. Data dependent detection of MS2 spectra was performed at a normalized collision energy of 30 % and an activation Time of 10 ms with an automatic definition of the scan range and a mass resolution (MS2) of 120000 using the orbitrap mass analyzer. Putative structures for the CoAs were generated using knowledge on the biology of the system. For 2-HMB-CoA and 2-EHB-CoA The fragment masses were obtained by inspecting the data files using Xcalibur Qual Browser and Freestyle software. The experimental fragments were used to verify the proposed structure of the molecules by drawing fragment structures to explain the observed fragment mass. The structures of the fragments were drawn using Chemdraw. All precursor ion and fragment ion masses differed by less than 5ppm compared to the theoretical mass.

**Computer simulations**

## Claims

1. A method for preparing an α-branched β'-hydroxy carbonyl compound by reductive aldol reaction, wherein the method comprises the steps:
a) providing an α,β-unsaturated carbonyl donor, wherein the α,β-unsaturated carbonyl donor is a coenzyme A thioester of an α,β-unsaturated carboxylic acid;
b) providing a carbonyl acceptor, wherein the carbonyl acceptor is an aldehyde;
c) performing an enzymatic-catalyzed reductive aldol reaction with the α,β-unsaturated carbonyl donor and the carbonyl acceptor by using a polypeptide capable of catalyzing reductive aldol reactions in the presence of a cofactor, wherein the polypeptide is an enoyl-CoA carboxylase/reductase.

2. The method according to claim 1, wherein the polypeptide is a crotonyl-CoA carboxylase/reductase.

3. The method according to claim 1 or 2, wherein the polypeptide comprises at least 95% sequence identity to amino acid sequence SEQ ID NO: 1 or SEQ ID NO:2.

4. The method according to any one of the claims 1 - 3, wherein the polypeptide comprises the amino acid sequence SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 or SEQ ID NO: 29.

5. The method according to any one of the claims 1 - 4, wherein the polypeptide is a wild type crotonyl-CoA carboxylase/reductase.

6. The method according to any one of the claims 1 - 5, wherein the polypeptide is a crotonyl-CoA carboxylase/reductase obtained from *Kitasatospora setae* or *Caulobacter crescentus.*

7. The method according to any one of the claims 1 - 6, wherein the cofactor is NADPH.

8. The method according to any one of the claims 1 - 7, wherein the α,β-unsaturated carboxylic acid has the general formula (**I**): 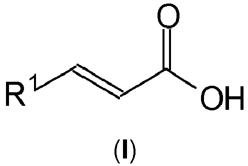 wherein R¹ is selected from hydrogen, an optionally substituted alkyl, alkenyl, alkynyl, alkoxy, carboxy, aminocarbonyl, thiocarbonyl, heteroalkyl, heteroalkenyl, heteroalkynyl, carboxyalkyl, aminoalkyl, haloalkyl, alkylthioalkyl, cycloalkyl, aryl, arylalkyl, heterocycloalkyl, heteroaryl, and heteroarylalkyl.

9. The method according to claim 8, wherein
R¹ represents -H, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -C₂H₄Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -C₇H₁₅, -C₈H₁₇, -C(CH₃)₂-C₃H₇, -CH(CH₃)-CH(CH₃)-C₂H₅, -CH₂-CH(CH₃)-CH(CH₃)₂, -CH₂-C(CH₃)₂-C₂H₅, -CH(CH₃)-C(CH₃)₃, -C(CH₃)₂-CH(CH₃)₂, -C₂H₄-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -CH₂-C(CH₃)=CH₂, -CH(CH₃)-CH=CH, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₂H₄-CH=CH₂, -CH=CH-C₂H₅, -CH=C(CH₃)₂, -CH₂-CH=CH-CH₃, -CH=CH-CH=CH₂, -C₃H₆-CH=CH₂, -CH=CH-C₃H₇, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -CH₂-C=CH, -C=CH, -C=C-CH₃, -C₂H₄-C=CH, -C=C-C₂H₅, -CH₂-C=C-CH₃, -C=C-CH=CH₂, -CH=CH-C=CH, -C=C-C=CH, -C₃H₆-C=CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₄Hg-C≡CH, -C≡C-C₄H₉, -C₃H₆-C≡C-CH₃, -CH₂-C≡C-C₃H₇, -C₂H₄Ph, -CH=CH-Ph, -C=C-Ph, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -CH₂R², -CH₂CH₂R², or -CH₂CH₂CH₂R²; and R² represents -NH₂, -OH, -SH, -F, -CI, -Br, -I, -CN, -N₃, -OCN, -NCO, -SCN, or -NCS.

10. The method according to any one of the claims 1 - 9, wherein the carbonyl acceptor has the general formula (**II**): 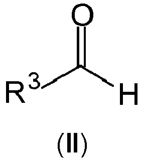 wherein R³ represents -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -Ph, or -CH₂-Ph.

11. The method according to any one of the Claims 1 - 10, wherein the carbonyl acceptor is selected from the group comprising or consisting of formaldehyde, acetaldehyde and propionaldehyde.

12. The method according to any one of the Claims 1 - 11, wherein the α,β-unsaturated carboxylic acid is selected from the group comprising or consisting of crotonic acid (*trans*-2-butenoic acid), *trans*-cinnamic acid, 5-chloro-2-pentenoic acid, *trans*-2-hexenoic acid, 5-methyl-2-hexenoic acid, *trans*-2-penten-4-ynoic acid and penta-2,4-dienoic acid.

13. The method according to any one of the Claims 1 - 12, wherein the enzyme-catalyzed reductive aldol reaction is performed in the presence of an acyl-CoA oxidase.

14. The method according to any one of the Claims 1 - 13, further comprising the following step:
d) performing a hydrolysis reaction with the coenzyme A thioester of the α-branched β'-hydroxy carbonyl compound under basic conditions or by using a thioesterase.

15. The method according to any one of the Claims 1 - 14, wherein the α-branched β'-hydroxy carbonyl compound has the general formula (**IIIa**) and the α-branched β'-hydroxy acyl-CoA has the general formula (**IIIb**): 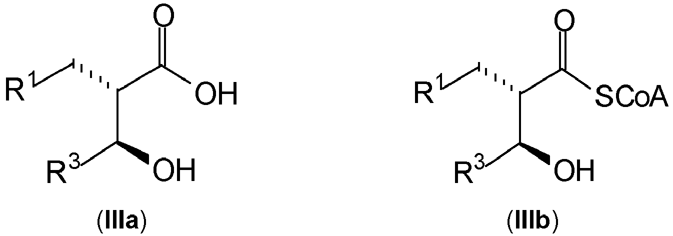 and R¹, R², and R³ have the same meanings as defined in any one of the claims 8 - 10.
